# EUROPEAN PATENT APPLICATION

(11) **EP 2 896 348 A1**
(43) Date of publication of application: **22.07.2015**
(21) Application number: 13836596.0
(22) Date of filing: 11.04.2013
(51) Int. Cl.: A61B 1/00

(54) **ENDOSCOPE CLEANING SHEATH AND ENDOSCOPE DEVICE**

(30) Priority: 14.09.2012 JP 2012202943
(71) Applicant: Olympus Medical Systems Corp., Tokyo 151-0072 (JP)
(72) Inventor: MIYAMOTO, Shinichi, Hachioji-shi Tokyo 192-8512 (JP); FUJIMOTO, Ryuhei, Hachioji-shi Tokyo 192-8512 (JP); SHINTANI, Keishiro, Hachioji-shi Tokyo 192-8512 (JP)
(74) Representative: Schicker, Silvia
(86) International application number: PCT/JP2013/060954
(87) International publication number: WO 2014/041837

(57) **Abstract**

An endoscope cleaning sheath includes a sheath main body, a distal-side fixing portion fixed to a distal portion of the sheath main body, and a proximal-side fixing portion fixed to a proximal portion of the sheath main body. The endoscope cleaning sheath includes a linear portion having a distal end fixed to the distal-side fixing portion at a distal-side connecting position and a proximal end fixed to the proximal-side fixing portion at a proximal-side connecting position, extended along a linear core axis between the distal-side connecting position and the proximal-side connecting position, and having a higher tensile strength in directions parallel to the longitudinal axis than the sheath main body.

## Description

### Technical Field

The present invention relates to an endoscope cleaning sheath attached to an endoscope which includes an observation window provided at a distal portion of an insertion section, and to an endoscope device including the endoscope cleaning sheath.

### Background Art

Patent Literature 1 discloses an endoscope cleaning sheath attached to an endoscope which includes an observation window provided in a distal portion of an insertion section. This endoscope cleaning sheath includes a multi-lumen tube that is a sheath main body. In the multi-lumen tube, an endoscope insertion duct into which the insertion section of the endoscope can be inserted is defined along a longitudinal axis. Further, in the multi-lumen tube, an air supply duct through which air can be supplied as a fluid from a proximal direction toward a distal direction and a water supply duct through which water can be supplied as a fluid from the proximal direction toward the distal direction are defined as fluid ducts. A distal-side fixing portion is fixed to a distal portion of the multi-lumen tube. In the distal-side fixing portion, a merging portion where the air supply duct and the water supply duct are merged and a nozzle as a fluid emitting portion that can emit air and water merged in the merging portion are provided. In a state that the endoscope cleaning sheath is attached to an endoscope, the merged air and water can be emitted from the nozzle toward the observation window of the insertion section. When the air and the water are emitted toward the observation window, contamination adhering to the observation window can be cleaned off without removing the insertion section from a body cavity in observation of a subject using the endoscope in the body cavity.

Patent literature 2 also discloses an endoscope cleaning sheath attached to an endoscope which includes an observation window provided at a distal portion of an insertion section. In this endoscope cleaning sheath are also provided with a multi-lumen tube as a sheath main body and a distal-side fixing portion. Moreover, in the multi-lumen tube, an endoscope insertion duct, an air supply duct, and a water supply duct are provided. Additionally, at the distal-side fixing portion, a merging portion for the air supply duct and the water supply duct and a nozzle as a fluid emitting portion from which merged air and water can be emitted toward the observation window of the insertion section are provided. In this endoscope cleaning sheath is provided with a wiper that can wipe the observation window of the insertion section when the endoscope cleaning sheath is attached to the endoscope. A distal end of a wire is connected to the wiper. A wire duct communicating with the merging portion is provided in the multi-lumen tube. Further, a proximal-side fixing portion is fixed at a proximal portion of the multi-lumen tube. The wire is passed through the merging portion and the wire duct, and a proximal end of the wire is connected to the proximal-side fixing portion through a tension spring. In a state that the air and the water are not emitted from the nozzle, the wiper is placed between the nozzle and the observation window by an energizing force of the tension spring, and the wiper is outside of a viewing field that is provided through the observation window. On the other hand, in a state that the air and the water are emitted from the nozzle, the wiper moves toward the observation window due to an emission pressure against the energizing force. As a result, the observation window is wiped by the wiper.

### Citation List

### Patent Literatures

Patent Literature 1: Jpn. Pat. Appln. KOKAI Publication No. 2009-247566
Patent Literature 2: Jpn. Pat. Appln. KOKAI Publication No. 2008-279202

### Summary of Invention

### Technical Problem

There is a case that an endoscope cleaning sheath like those in Patent Literature 1 and Patent Literature 2 is attached to an endoscope having a bending section provided in an insertion section. In this case, the multi-lumen tube as a sheath main body must be configured to be bendable in accordance with a bending motion of the bending section. Therefore, the multi-lumen tube is made of a soft material such as silicone and has flexibility. However, there is a case that, during the use of an endoscope device including an endoscope cleaning sheath, an excessive external force acts on the multi-lumen tube. In this case, the multi-lumen is pulled in directions parallel to a longitudinal axis. Since the multi-lumen tube is made of a soft material, a tensile strength of the endoscope cleaning sheath in the directions parallel to the longitudinal axis is low. Therefore, when the multi-lumen tube (the sheath main body) is pulled in the directions parallel to the longitudinal axis, the multi-lumen tube is expanded, and the endoscope cleaning sheath may possibly be damaged.

In view of the above-described problem, it is an object of the present invention to provide an endoscope cleaning sheath whose tensile strength in directions parallel to a longitudinal axis is assured. Furthermore, it is another object of the present invention to provide an endoscope device including the endoscope cleaning sheath.

### Solution to Problem

To solve above mentioned problems, according to one aspect of the invention, an endoscope cleaning sheath attached to an endoscope which includes an insertion section extended along a longitudinal axis, and in which an observation window is provided to a distal portion of the insertion section, the endoscope cleaning sheath including that: a sheath main body including: an insertion duct defining portion defining an endoscope insertion duct, which enables the insertion section of the endoscope to be inserted therein, along the longitudinal axis; and a fluid duct defining portion defining a fluid duct which enables a fluid to be supplied from a proximal direction toward a distal direction; a distal-side fixing portion which is fixed to a distal portion of the sheath main body, and which includes a fluid emitting portion enabling emission of the fluid having passed through the fluid duct toward the observation window of the endoscope in a state that the endoscope cleaning sheath is attached to the endoscope; a proximal-side fixing portion which is fixed to a proximal portion of the sheath main body; and a linear portion which has a distal end fixed to the distal-side fixing portion at a distal-side connecting position, and a proximal end fixed to the proximal-side fixing portion at a proximal-side connecting position, and which is extended along a linear core axis between the distal-side connecting position and the proximal-side connecting position, the linear portion having a higher tensile strength in directions parallel to the longitudinal axis than the sheath main body.

### Advantageous Effects of Invention

According to the present invention, the endoscope cleaning sheath whose tensile strength in the directions parallel to the longitudinal axis is assured can be provided. Moreover, the endoscope device including the endoscope cleaning sheath can be provided.

### Brief Description of Drawings

FIG. 1 is a schematic view showing a configuration of an endoscope device according to a first embodiment of the present invention;
FIG. 2 is a partial side-elevation cross-sectional view schematically showing a configuration of an endoscope cleaning sheath according to the first embodiment;
FIG. 3 is a cross-sectional view taken along a line III-III in FIG. 2;
FIG. 4 is a schematic view showing configurations of a distal-side fixing portion and a distal main body section in a state that the endoscope cleaning sheath is attached to the endoscope according to the first embodiment;
FIG. 5 is a cross-sectional view schematically showing configurations of a multi-lumen tube and a linear portion near given one of first holes and given one of second holes in a state that the multi-lumen tube according to the first embodiment is not expanded in directions parallel to the longitudinal axis;
FIG. 6 is a schematic view showing a state that a distal surface of an insertion section of an endoscope is placed to a proximal direction side with respect to the given one of the first holes in attachment of the endoscope cleaning sheath to the endoscope according to the first embodiment;
FIG. 7 is a schematic view showing a state that the distal surface of the insertion section of the endoscope has moved up to the given one of the first holes in the directions parallel to the longitudinal axis in the attachment of the endoscope cleaning sheath to the endoscope according to the first embodiment;
FIG. 8 is a schematic view showing a state that the distal surface of the insertion section is placed to a distal direction side with respect to the given one of the second holes in the attachment of the endoscope cleaning sheath to the endoscope according to the first embodiment;
FIG. 9 is a schematic view showing a state that the distal surface of the insertion section of the endoscope has moved to the distal direction side from the second hole placed most distally in the attachment of the endoscope cleaning sheath to the endoscope according to the first embodiment;
FIG. 10A is a schematic view showing a state that the distal surface of the insertion section of the endoscope has moved to the distal direction side from the second hole placed most distally in the attachment of the endoscope cleaning sheath to the endoscope according to a first modification;
FIG. 10B is a cross-sectional view schematically showing the configurations of the multi-lumen tube and the linear portion near the given one of the first holes and the given one of the second holes in a state that the multi-lumen tube according to the first embodiment is expanded in the directions parallel to the longitudinal axis;
FIG. 11 is a cross-sectional view schematically showing the configurations of the multi-lumen tube and the linear portion near the given one of the first holes and the given one of the second holes according to a second modification;
FIG. 12 is a cross-sectional view schematically showing the configurations of the multi-lumen tube and the linear portion near the given one of the first holes and the given one of the second holes according to a third modification;
FIG. 13 is a cross-sectional view schematically showing a configuration of the endoscope cleaning sheath according to a fourth modification;
FIG. 14A is a cross-sectional view schematically showing an internal configuration of the multi-lumen tube of the endoscope cleaning sheath according to a fifth modification;
FIG. 14B is a cross-sectional view schematically showing the internal configuration of the multi-lumen tube of the endoscope cleaning sheath according to a sixth modification;
FIG. 15A is a cross-sectional view schematically showing the configuration of the endoscope cleaning sheath according to a seventh modification;
FIG. 15B is a schematic view showing a distal-side fixing portion of the endoscope cleaning sheath according to an eighth modification viewed from the distal direction side;
FIG. 16 is a schematic view schematically showing a configuration of an endoscope cleaning sheath according to a second embodiment;
FIG. 17 is a perspective view schematically showing configurations of a multi-lumen tube and a linear portion near a given one of holes of the endoscope cleaning sheath according to the second embodiment;
FIG. 18 is a cross-sectional view schematically showing the configurations of the multi-lumen tube and the linear portion near the given one of the holes of the endoscope cleaning sheath according to the second embodiment;
FIG. 19A is a schematic view showing an extended state of the linear portion near the given one of the holes in a state that the endoscope cleaning sheath is attached to an endoscope according to the second embodiment;
FIG. 19B is a schematic view showing an extended state of the linear portion near the given one of the holes in a state that an insertion section has moved toward a proximal direction in an endoscope insertion duct according to the second embodiment;
FIG. 20 is a perspective view schematically showing the configuration of the endoscope cleaning sheath according to a ninth modification;
FIG. 21 is a perspective view schematically showing a configuration of an endoscope cleaning sheath according to a third embodiment;
FIG. 22 is a perspective view schematically showing the configuration of the endoscope cleaning sheath according to a 10th modification;
FIG. 23 is a cross-sectional view schematically showing an internal configuration of a multi-lumen tube of an endoscope cleaning sheath according to a fourth embodiment;
FIG. 24 is a cross-sectional view schematically showing an internal configuration of a multi-lumen tube of an endoscope cleaning sheath according to a fifth embodiment;
FIG. 25 is a cross-sectional view schematically showing a configuration of an endoscope cleaning sheath according to a sixth embodiment;
FIG. 26 is a cross-sectional view taken along a line 26-26 in FIG. 25;
FIG. 27 is a cross-sectional view taken along a line 27-27 in FIG. 25;
FIG. 28 is a cross-sectional view schematically showing configurations of a distal portion of an endoscope and a distal portion of the endoscope cleaning sheath in a state that the endoscope cleaning sheath is attached to the endoscope according to the sixth embodiment;
FIG. 29 is a cross-sectional view schematically showing the configuration of the distal portion of the endoscope cleaning sheath according to an 11th modification;
FIG. 30 is a cross-sectional view schematically showing the configuration of the distal portion of the endoscope cleaning sheath according to a 12th modification;
FIG. 31 is a cross-sectional view schematically showing the configuration of the distal portion of the endoscope cleaning sheath according to a 13th modification; and
FIG. 32 is a cross-sectional view schematically showing the configuration of the distal portion of the endoscope cleaning sheath according to 14th modification. Description of Embodiments

### (First Embodiment)

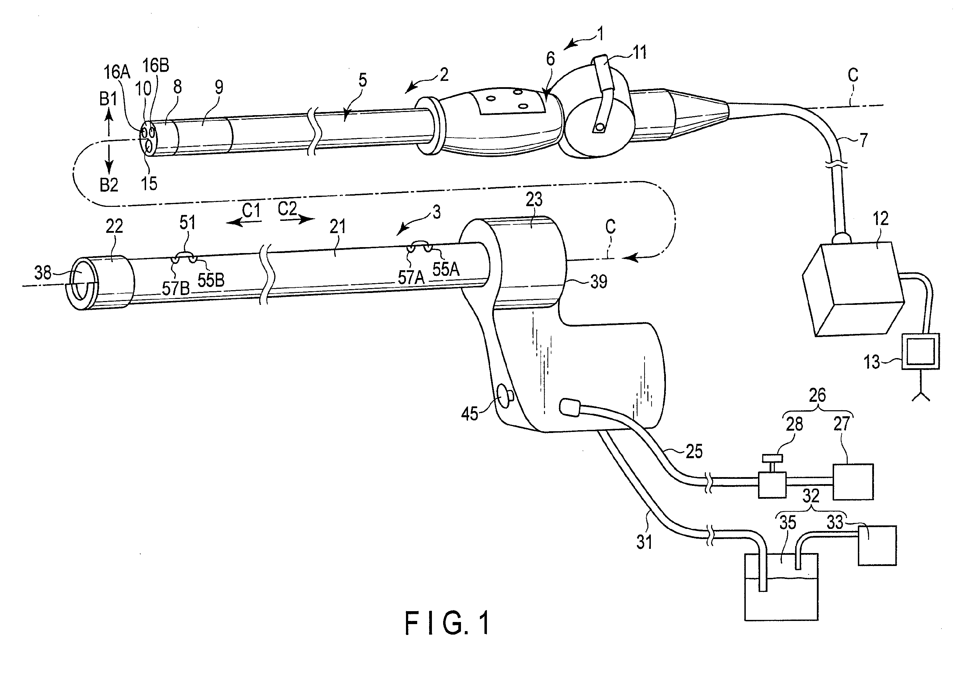

A first embodiment according to the present invention will now be described with reference to FIG. 1 to FIG. 10B.

FIG. 1 is a view showing a configuration of an endoscope device 1. As shown in FIG. 1, the endoscope device 1 has a longitudinal axis C. One of directions parallel to the longitudinal axis C is a distal direction (a direction of an arrow C in FIG. 1), and an opposite direction of the distal direction is a proximal direction (a direction of an arrow C2 in FIG. 1). The endoscope apparatus 1 includes an endoscope 2 and an endoscope cleaning sheath 3 attached to the endoscope 2.

The endoscope 2 is, e.g., a rigid endoscope. The endoscope 2 includes an insertion section 5 which is extended along the longitudinal axis C, and an operation section 6 which is provided to the proximal direction side with respect to the insertion section 5. One end of a universal cord 7 is connected to the operation section 6. The insertion section 5 includes a distal main body section 8 which forms a distal surface 10 of the insertion section 5, and a bending section 9 which is provided to the proximal direction side with respect to the distal main body section 8. The bending section 9 is provided in a distal portion of the insertion section 5 and can bend in two directions perpendicular to the longitudinal axis C. That is, the direction of the arrow B1 and the direction of the arrow B2 in FIG. 1 are bending directions toward which the bending section 9 can bend. It is to be noted that the bending section 9 can bend in the two directions perpendicular to the longitudinal axis C in this embodiment, but the bending section 9 may be capable of bending in four directions perpendicular to the longitudinal axis C. A bending operation lever 11 that is a bending operation section by which operations for bending the bending section 9 are input is provided to the operation section 6.

The other end of the universal cord 7 is connected to an observation control unit 12. The observation control unit 12 includes an image processing section (not shown) such as an image processor and a light source section (not shown). The image processing section of the observation control unit 12 is electrically connected to a monitor 13 as a display section. An observation window 15 and illumination windows 16A and 16B are provided to the distal surface 10 (the distal portion) of the insertion section 5. Two light guides (not shown) are extended in the insertion section 5 along the longitudinal axis C. The light guides are optically connected to the light source section of the observation control unit 12 through an inside of the operation section 6 and an inside of the universal cord 7. Light emitted from the light source section is guided by the light guides. Further, a subject is irradiated with the guided light through the illumination windows 16A and 16B.

An imaging element (not shown) such as a CCD is provided in the distal main body portion 8. The imaging element is configured to image the subject. One end of an imaging cable (not shown) is connected to the imaging element. The other end of the imaging cable is connected to the image processing section of the observation control unit 12 through the inside of the insertion section 5, the inside of the operation section 6, and the inside of the universal cord 7. When the subject is imaged by the imaging element and an electrical signal is transmitted to the image processing section through the imaging cable, image processing is executed in the image processing section, and an image of the subject is generated. The generated image of the subject is displayed on the monitor 13.

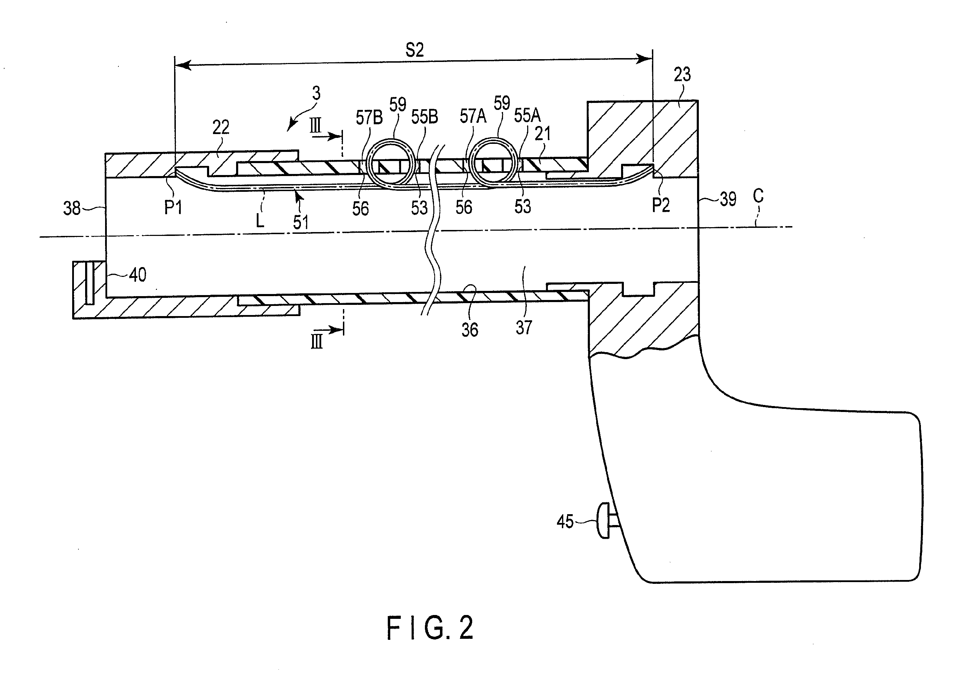

FIG. 2 is a view showing a configuration of the endoscope cleaning sheath 3. As shown in FIG. 1 and FIG. 2, the endoscope cleaning sheath 3 includes a multi-lumen tube 21 that is a sheath main body extended along the longitudinal axis C. The multi-lumen tube 21 is made of a soft material such as silicone or urethane and has flexibility. Therefore, the multi-lumen 21 can bend in accordance with bending moion of the bending section 9 of the insertion section 5.

A distal-side fixing portion 22 is fixed to a distal portion of the multi-lumen tube 21. Furthermore, a proximal-side fixing portion 23 is fixed to a proximal portion of the multi-lumen tube 21. The distal-side fixing portion 22 and the proximal-side fixing portion 23 are made of a harder material than the multi-lumen tube 21 and do not have the flexibility.

One end of an air supply tube 25 is connected to the proximal-side fixing portion 23. The other end of the air supply tube 25 is connected to an air supply unit 26. The air supply unit 26 includes an air supply pump 27 and a pressure adjustment valve 28. When the air supply pump 27 is driven, air as a fluid is supplied through the air supply tube 25 by an adjusted pressure.

One end of a water supply tube 31 is connected to the proximal-side fixing portion 23. The other end of the water supply tube 31 is connected to a water supply unit 32. The water supply unit 32 includes a water supply pump 33 and a water supply tank 35. When the water supply pump 33 is driven, water as a fluid is supplied from the water supply tank 35 through the water supply tube 31.

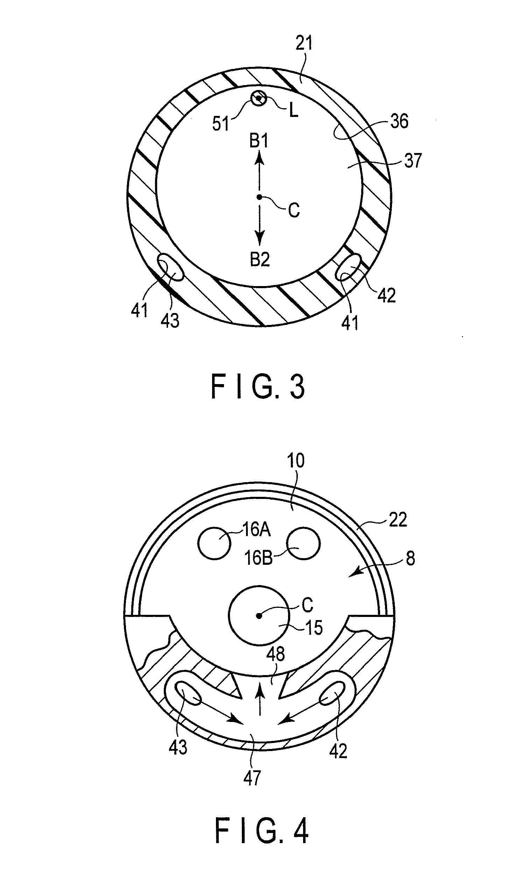

FIG. 3 is a cross-sectional view taken along a line III-III in FIG. 2. As shown in FIG. 2 and FIG. 3, in the multi-lumen tube 21, an endoscope insertion duct 37 is defined along the longitudinal axis C by an insertion duct defining portion 36. The insertion section 5 of the endoscope 2 can be inserted into the endoscope insertion duct 37. In the distal-side fixing portion 22, a distal-side opening portion 38 communicating with the endoscope insertion conduit 37 is provided. Moreover, in the proximal-side fixing portion 23, a proximal-side opening portion 39 communicating with the endoscope insertion duct 37 is provided. When the insertion section 5 is inserted into the endoscope insertion conduit 37 from the proximal-side opening portion 39, the endoscope cleaning sheath 3 is attached to the endoscope 2. Additionally, an abutting portion 40 on which the distal plane 10 of the insertion section 5 can abut is provided to the distal-side fixing portion 22. In a state that the endoscope cleaning sheath 3 is attached to the endoscope 2, the abutting portion 40 abuts on the distal surface 10 of the insertion section 5.

As shown in FIG. 3, in the multi-lumen tube 21, an air supply duct 42 is defined along the longitudinal axis C by a fluid duct defining portion 41. Further, in the multi-lumen tube 21, a water supply duct 43 is defined along the longitudinal axis C by the fluid duct defining portion 41. The air supply conduit 42 and the water supply conduit 43 are defined to be apart from each other. Furthermore, the air supply duct 42 and the water supply duct 43 are separated from the endoscope insertion duct 37 by the insertion duct defining portion 36 and the fluid duct defining portion 41. It is to be noted that, in a state that the endoscope cleaning sheath 3 is attached to the endoscope 2, a direction of an arrow B1 and a direction of an arrow B2 in FIG. 3 are bending directions of the bending section 9 of the endoscope 2. Therefore, each of the air supply duct 42 and the water supply duct 43 is placed to be approximately 135° apart from one of the bending directions (the direction of the arrow B1) of the bending section 9 in directions around the longitudinal axis. Moreover, each of the air supply duct 42 and the water supply duct 43 is placed to be approximately 45° apart from the other of the bending directions (the direction of the arrow B2) of the bending section 9 in the directions around the longitudinal axis. That is, each of the air supply conduit 42 and the water supply conduit 43 is placed at an angular position deviating from each bending direction of the bending section 9 in the directions around the longitudinal axis.

An air supply intermediary portion (not shown) that intermediates between an inside of the air supply tube 25 and the air supply duct 42 is provided to the proximal-side fixing portion 23. An air supply control valve (not shown) is provided to the air supply intermediary portion. Additionally, a water supply intermediary portion (not shown) that intermediates between an inside of the water supply tube 31 and the water supply duct 43 is provided to the proximal-side fixing portion 23. A water supply control valve (not shown) is provided to the water supply intermediary portion.

Further, a control valve operation button 45 that is a control valve operation section is attached to the proximal-side fixing portion 23. When the control valve operation button 45 is pressed, the air supply intermediary portion is opened at the air supply control valve, and air is supplied to the air supply duct 42 from the air supply tube 25 through the air supply intermediary portion. Furthermore, in the air supply duct 42, the air is supplied from the proximal direction toward distal direction. Moreover, when the control valve operation button 45 is pressed, the water supply intermediary portion is opened at the water supply control valve, and water is supplied to the water supply duct 43 from the water supply tube 31 through the water supply intermediary portion. Additionally, in the water supply duct 43, the water is supplied from the proximal direction toward the distal direction. As described above, each of the air supply duct 42 and the water supply duct 43 is a fluid duct through which a fluid can be supplied from the proximal direction toward the distal direction.

FIG. 4 is a view showing configurations of the distal-side fixing portion 22 and the distal main body portion 8 in a state that the endoscope cleaning sheath 3 is attached to the endoscope 2. As shown in FIG. 4, a merging portion 47 in which the air supply duct 42 and the water supply duct 43 are merged is provided in the distal-side fixing portion 22. In the merging portion 47, the air that has passed through the air supply duct 42 and the water that has passed through the water supply duct 43 are merged. Moreover, a nozzle 48 that is a fluid emitting portion from which the air and the water merged in the merging portion 47 can be emitted is provided to the distal-side fixing portion 22. In a state that the endoscope cleaning sheath 3 is attached to the endoscope 2, the air and the water are emitted toward the observation window 15. That is, a fluid can be emitted from the nozzle 48 toward the observation window 15.

As shown in FIG. 2 and FIG. 3, a linear portion 51 is extended between the distal-side fixing portion 22 and the proximal-side fixing portion 23 through the endoscope insertion duct 37. A distal end of the linear portion 51 is fixed to the distal-side fixing portion 22 at a distal-side connecting position P1. Additionally, a proximal end of the linear portion 51 is fixed to the proximal-side fixing portion 23 at a proximal-side connecting position P2. The linear portion 51 is extended along a linear core axis L between the distal-side connecting position P1 and the proximal-side connecting position P2. The linear core axis L is a central axis of the linear portion 51. The linear portion 51 is made of a fiber such as an aramid fiber, a nylon fiber, a polyethylene fiber, or a polyarylate fiber, and a tensile strength in the directions parallel to the longitudinal axis C is higher than that of the multi-lumen tube 21. Therefore, the linear portion 51 has such resistant strength that the linear portion 51 is not expanded even when it is pulled in the directions parallel to the longitudinal axis C.

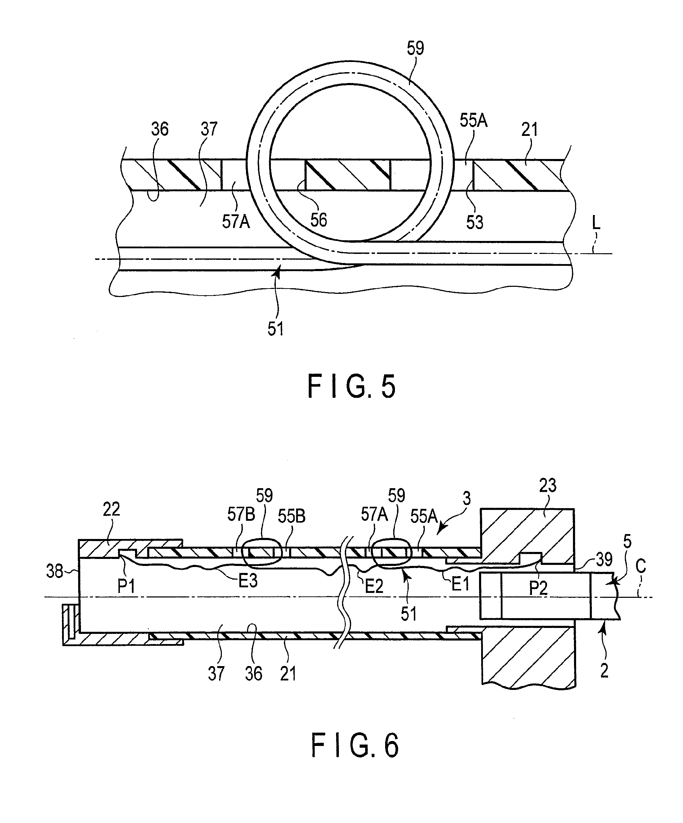

A linear dimension S1 of the linear portion 51 along the linear core axis L between the distal-side connecting position P1 and the proximal-side connecting position P2 is larger than a parallel axial dimension S2 along the longitudinal axis C between the distal-side connecting position P1 and the proximal-side connecting position P2. Further, the linear portion 51 is placed at substantially the same angular position as one of the bending directions (the direction of the arrow B1 in FIG. 3) of the bending section 9 of the endoscope 2 in the directions around the longitudinal axis. Furthermore, the distal-side connecting position P1 of the linear portion 51 is placed at an angular position that is substantially 180° apart from the nozzle 48 in the directions around the longitudinal axis. As shown in FIG. 1 and FIG. 2, in the multi-lumen tube 21 as a sheath main body, first holes 55A and 55B are defined by a first hole defining portion 53, and second holes 57A and 57B different from the first holes 55A and 55B are defined by a second hole defining portion 56. The first hole 55A is placed to the proximal direction side with respect to the first hole 55B. Moreover, the second hole 57A is placed to the proximal direction side with respect to the second hole 57B. FIG. 5 is a view showing configurations of the multi-lumen tube 21 and the linear portion 51 near a given one 55A of the first holes and a given one 57A of the second holes. It is to be noted that a configuration near the first hole 55A and the second hole 57A alone will be described hereinafter and the same description will be given as to a configuration near the first hole 55B and the second hole 57B. Additionally, in this embodiment, the two first holes 55A and 55B and the two second holes 57A and 57B are provided, and defining one or more first holes (55A and 55B) and one or more second holes (57A and 57B) in the multi-lumen tube 21 can suffice.

As shown in FIG. 5, the endoscope insertion duct 37 communicates with an outside of the multi-lumen tube 21 through the first hole 55A. Further, the endoscope insertion duct 37 communicates with the outside of the multi-lumen tube 21 through the second hole 57A. The first hole 55A is placed to the proximal direction side with respect to the second hole 57A. That is, the first hole 55A and the second hole 57A are apart from each other in the directions parallel to the longitudinal axis C.

In the endoscope insertion duct 37, the linear portion 51 is extended toward the proximaldirection until it reaches the first hole 55A. Furthermore, in the first hole 55A, the linear portion 51 is extended from the endoscope insertion duct 37 to the outside of the multi-lumen tube 21. Moreover, on an outer peripheral portion of the multi-lumen tube 21, the linear portion 51 is extended from the first hole 55A toward the distal direction until it reaches the second hole 57A. Additionally, in the second hole 57A, the linear portion 51 is inserted into the endoscope insertion conduit 37 from the outside of the multi-lumen tube 21. Further, in the endoscope insertion duct 37, the linear portion 51 is extended from the second hole 57A toward the proximal direction. When the linear portion 51 is extended as described above, a loop-shaped portion 59 is formed in the linear portion 51 between the first hole 55A and the second hole 57A.

Functions and effects of the endoscope device 1 according to this embodiment will now be described. In the endoscope device 1, the insertion section 5 and the endoscope cleaning sheath 3 are inserted into a body cavity in a state that the endoscope cleaning sheath 3 is attached to the endoscope 2. Furthermore, a subject is imaged by the imaging element (not shown) through the observation window 15. If contamination adheres to the observation window 15, the control valve operation button 45 is pressed. As a result, air is supplied from the proximal direction toward the distal direction in the air supply duct 42, and water is supplied from the proximal direction toward the distal direction in the water supply duct 43. Moreover, the air and the water are merged in the merging portion 47, and the air and the water are emitted from the nozzle 48 toward the observation window 15. As a result, the contamination adhering to the observation window 15 is cleaned off without removing the insertion section 5 and the endoscope cleaning sheath 3 from the body cavity.

Here, in observation of a subject using the endoscope apparatus 1, there are cases that an excessive external force acts on the multi-lumen tube 21. In this case, the multi-lumen tube 21 is pulled in the directions parallel to the longitudinal axis C. The distal-side fixing portion 22 and the proximal-side fixing portion 23 are fixed to the multi-lumen tube 21. Additionally, the distal end of the linear portion 51 is fixed to the distal-side fixing portion 22, and the proximal end of the linear portion 51 is fixed to the proximal-side fixing portion 23. The linear portion 51 has a higher tensile strength in the directions parallel to the longitudinal axis C than the multi-lumen tube 21, and the linear portion 51 is not expanded even if it is pulled in the directions parallel to the longitudinal axis C. When the linear portion 51 is provided, the tensile strength of the multi-lumen tube 21 in the directions parallel to the longitudinal axis C increases. Therefore, it is possible to effectively prevent the endoscope cleaning sheath 3 from being damaged due to pulling in the directions parallel to the longitudinal axis C.

As described above, when the linear portion 51 is provided, the tensile strength of the multi-lumen tube 21 in the directions parallel to the longitudinal axis C increases. However, when the multi-lumen tube 21 is pulled in the directions parallel to the longitudinal axis C by external force, the multi-lumen tube 21 expands in the directions parallel to the longitudinal axis C to some extent. Further, when the multi-lumen tube 21 as the sheath main body bends in accordance with a bending motion of the bending section 9, the multi-lumen tube 21 is likewise pulled in the directions parallel to the longitudinal axis C in a bending outer side part. In this case, likewise, the multi-lumen tube 21 expands in the directions parallel to the longitudinal axis C. Furthermore, in a state that the endoscope cleaning sheath 3 is attached to the endoscope 2, the distal surface 10 of the insertion section 5 must be arranged to closely abut on the abutting portion 40 of the distal-side fixing portion 22. Therefore, in a state that the endoscope cleaning sheath 3 is attached to the endoscope 2, the multi-lumen tube 21 expands in the directions parallel to the longitudinal axis C as compared with a case that the endoscope cleaning sheath 3 is not attacned to the endoscope 2.

The linear dimension S1 of the linear portion 51 along the linear core axis L between the distal-side connecting position P1 and the proximal-side connecting position P2 is larger than the parallel axial dimension S2 along the longitudinal axis C between the distal-side connecting position P1 and the proximal-side connecting position P2. That is, slack is generated in the linear portion 51. When the slack is generated in the linear portion 51, expansion of the multi-lumen tube 21 is not inhibited by the linear portion 51 even if the multi-lumen tube expands in the directions parallel to the longitudinal axis C. Therefore, when the multi-lumen tube 21 bends in accordance with a bending motion of the bending section 9, the bending motion of the bending section 9 is not inhibited by the linear portion 51. Therefore, even if the linear portion 51 that is not expanded by pulling in the directions parallel to the longitudinal axis C is provided, bendability of the bending section 9 is assured. Moreover, even in a case of attaching the endoscope cleaning sheath 3 to the endoscope 2, the expansion of the multi-lumen tube 21 is not inhibited by the linear portion 51. Therefore, operability for attaching the endoscope cleaning sheath 3 to the endoscope 2 is improved.

A method for attaching the endoscope cleaning sheath 3 to the endoscope 2 will now be described. As described above, in this embodiment, since the stretch of the multi-lumen tube 21 is not inhibited by the linear portion 51, the slack is generated in the linear portion 51. That is, the slack is generated in the linear portion 51 in the endoscope insertion duct 37. FIG. 6 is a view showing a state that the distal plane 10 of the insertion section 5 of the endoscope 2 is placed to the proximal direction side with respect to the given one 55A of the first holes in attachment of the endoscope cleaning sheath 3 to the endoscope 2. As shown in FIG. 6, in a case of attaching the endoscope cleaning sheath 3 to the endoscope 2, the insertion section 5 is inserted into the endoscope insertion conduit 37 from the proximal-side opening portion 39. Here, slack parts E1 to E3 are produced in the linear portion 51 in the endoscope insertion duct 37. In a state that the distal surface 10 of the insertion section 5 moves from a position on the proximal direction side with respect to the first hole 55A in the endoscope insertion duct 37 toward the distal direction, the slack part E1 placed to the proximal direction side with respect to the first hole 55A is pushed toward the distal direction by the distal surface 10 of the insertion section 5. As a result, the slack part E1 located to the proximal direction side of the first hole 55A moves toward the distal direction.

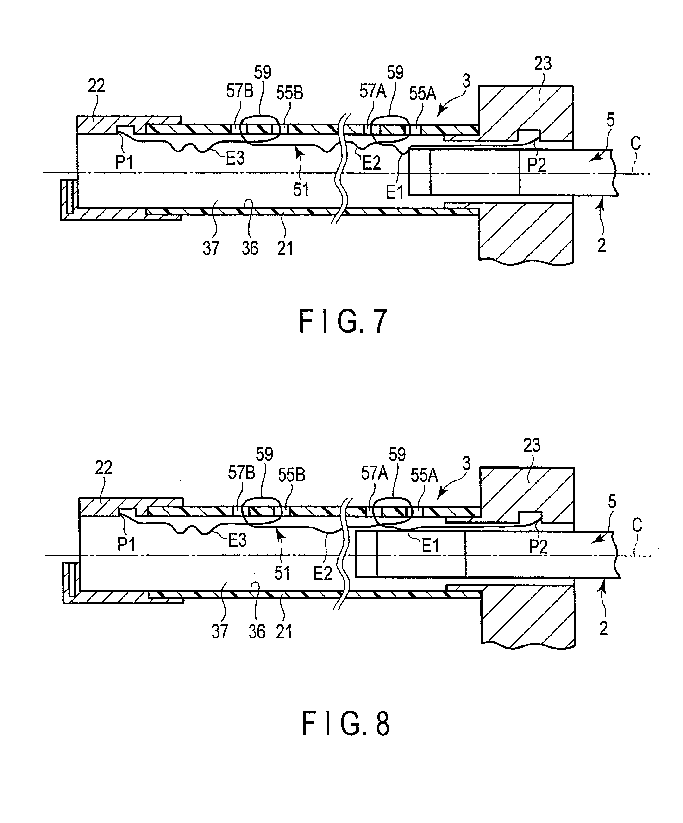

FIG. 7 is a view showing a state that the distal surface 10 of the insertion section 5 of the endoscope 2 has moved up to the given one 55A of the first holes in the directions parallel to the longitudinal axis C in the attachment of the endoscope cleaning sheath 3 to the endoscope 2. FIG. 8 is a view showing a state that the distal surface 10 of the insertion section 5 is placed to the distal direction side with respect to the given one 57A of the second holes in the attachment of the endoscope cleaning sheath 3 to the endoscope 2. As shown in FIG. 7, in the state that the distal plane 10 of the insertion section 5 of the endoscope 2 has moved up to the first hole 55A in the directions parallel to the longitudinal axis C, the slack part E1 located to the proximal direction side with respect to the first hole 55A moves toward the distal direction and located near the first hole 55A. Here, the linear portion 51 is extended on the outer peripheral portion of the multi-lumen tube 21 between the first hole 55A and the second hole 57A and forms the loop-shaped portion 59. Therefore, as shown in FIG. 8, even when the distal surface 10 of the insertion section 5 has moved to a region on the distal direction side with respect to the second hole 57A, the slack part E1 placed to the proximal direction side with respect to the first hole 55A does not move to the region on the distal direction side with respect to the second hole 57A.

Like the slack part E1 located to the proximal direction side with respect to the first hole 55A, the slack part E2 placed between the second hole 57A and the first hole 55B in the directions parallel to the longitudinal axis C is pushed toward the distal direction by the distal surface 10 of the insertion section 5. As a result, the slack part E2 placed between the second hole 57A and the first hole 55B moves toward the distal direction and located near the first hole 55B. Further, like the slack part E1 located to the proximal direction side with respect to the first hole 55A, even when the distal plane 10 of the insertion section 5 has moved to a region on the distal direction side with respect to the second hole 57B, the slack part E2 located between the second hole 57A and the first hole 55B does not move to the region on the distal direction side with respect to the second hole 57B.

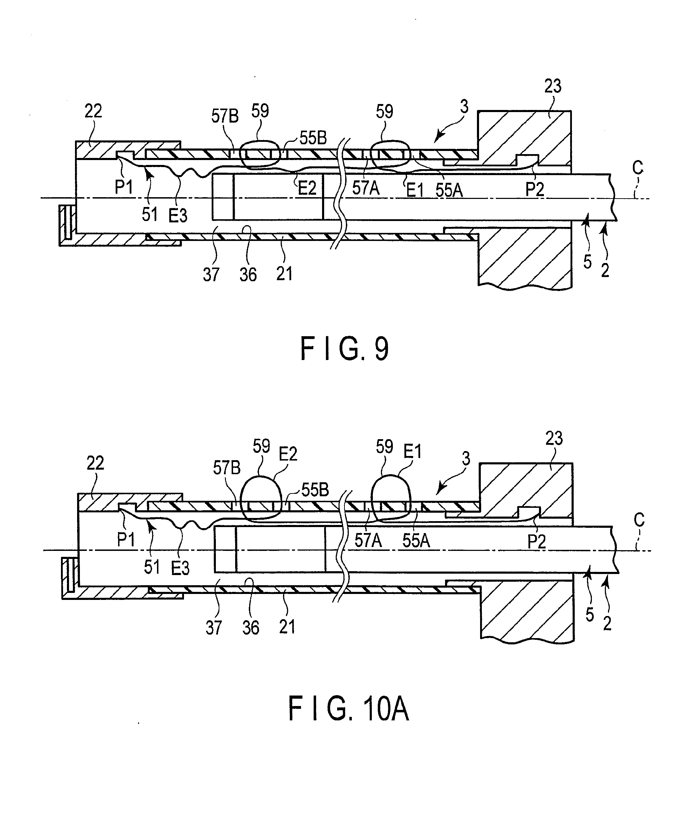

FIG. 9 is a view showing a state that the distal surface 10 of the insertion section 5 of the endoscope 2 has moved to the distal direction side from the second hole 57B, which is placed most distally among the second holes 57A and 57B, in the attachment of the endoscope cleaning sheath 3 to the endoscope 2. As described above, the slack part E1 located to the proximal direction side with respect to the first hole 55A does not move to the distal direction side from the second hole 57A, and the slack part E2 placed between the second hole 57A and the first hole 55B does not move to the distal direction side from the second hole 57B. Therefore, in a state that the distal surface 10 of the insertion section 5 has moved to a region on the distal direction side with respect to the second hole 57B, the slack parts E1 to E3 of the linear portion 51 are not dense in a part on the distal direction side with respect to the distal surface 10 of the insertion section 5, but instead are dispersed over the entire length of the endoscope insertion duct 37 in the directions parallel to the longitudinal axis C. That is, in a state that the distal surface 10 of the insertion section 5 has moved to the region to the distal direction side with respect to the second hole 57B, an amount of slack of the linear portion 51 does not increase in the part on the distal direction side with respect to the distal surface 10 of the insertion section 5. As a result, in a state that the insertion section 5 moves in the endoscope insertion duct 37 toward the distal direction, it is hard for the slack parts E1 to E3 of the linear portion 51 to be hitched on the insertion section 5. Therefore, even when the slack parts E1 to E3 are produced in the linear portion 51, insertability of the insertion section 5 in the endoscope insertion duct 37 can be assured.

It is to be noted that, in a first modification in which a rigidity of the linear portion 51 is changed from the first embodiment, the slack parts E1 to E3 move as follows. That is, as shown in FIG. 10A, when the slack part E1 is pushed by the distal surface 10 of the insertion section 5 in a state that the slack part E1 has moved toward the distal direction and located near the first hole 55A, the slack part E1 moves to a part of the linear portion 51 (the loop-shaped portion 59) extended on the outer peripheral portion of the multi-lumen tube 21 between the first hole 55A and the second hole 57A. When the slack part E1 moves to the outer peripheral portion of the multi-lumen tube 21 between the first hole 55A and the second hole 57A, the slack part E1 having been placed to the proximal direction side with respect to the first hole 55A is no longer pushed toward the distal direction by the distal plane 10 of the insertion section 5. As a result, even when the distal surface 10 of the insertion section 5 has moved to a region on the distal direction side with respect to the second hole 57A, the slack part E1 having been located to the proximal direction side with respect to the first hole 55A does not move to the distal direction side from the second hole 57A.

The slack part E2 located between the second hole 57A and the first hole 55B in the directions parallel to the longitudinal axis C behaves in the same manner as the slack part E1 located to the proximal end direction side with respect to the first hole 55A. That is, when the slack part E2 is pushed by the distal surface 10 of the insertion section 5 in a state that the slack part E2 has moved toward the distal direction and located near the first hole 55B, the slack part E2 moves to a part of the linear portion 51 (the loop-shaped portion 59) extended on the outer peripheral portion of the multi-lumen tube 21 between the first hole 55B and the second hole 57B. As a result, the slack part E2 having been located between the second hole 57A and the first hole 55B is no longer pushed toward the distal direction by the distal surface 10 of the insertion section 5. Therefore, even when the distal surface 10 of the insertion section 5 has moved to the region on the distal direction side with respect to the second hole 57B, the slack part E2 having been placed between the second hole 57A and the first hole 55B does not move to the distal direction side from the second hole 57B.

Therefore, in this modification, the slack parts E1 to E3 of the linear portion 51 do not densely form in a part on the distal direction side with respect to the distal surface 10 of the insertion section 5 when the distal surface 10 of the insertion section 5 has moved to the region to the distal direction side with respect to the second hole 57B. As a result, when the insertion section 5 moves toward the distal direction in the endoscope insertion duct 37, it is hard for the slack parts E1 to E3 of the linear portion 51 to be hitched on the insertion section 5. Therefore, even when the slack parts E1 to E3 are produced in the linear portion 51, the insertability of the insertion section 5 in the endoscope insertion duct 37 can be assured.

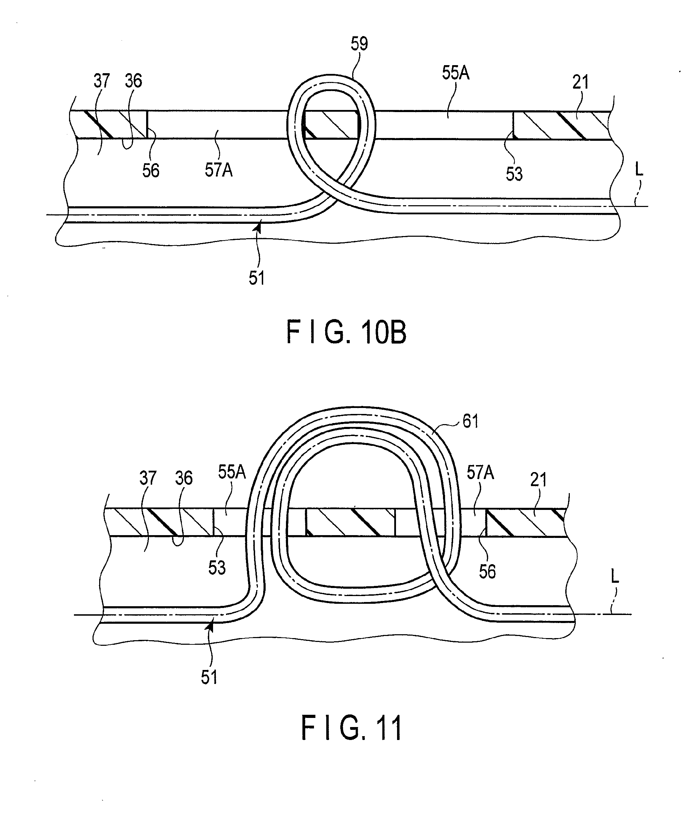

FIG. 10B is a view showing the configurations of the multi-lumen tube 21 and the linear portion 51 near a given one 55A of the first holes and a given one 57A of the second holes in a state that the multi-lumen tube 21 is expanded in the directions parallel to the longitudinal axis C. As shown in FIG. 10B, when the multi-lumen tube 21 is stretched in the directions parallel to the longitudinal axis C, the dimension of a part of the linear portion 51 other than the loop-shaped portion 59 along the linear core axis L increases between the distal-side connecting position P1 and the proximal-side connecting position P2 as compared with a state that the multi-lumen tube 21 is not stretched (see FIG. 5). Here, the linear portion 51 is not expanded by pulling in the directions parallel to the longitudinal axis C. Therefore, in the linear portion 51, since the dimension of the part other than the loop-shaped portion 59 along the linear core axis L increases, the dimension of the looped portion 59 along the linear core axis L decreases. That is, the linear portion 51 is deformed without being expanded in accordance with the expansion of the multi-lumen tube 21 in the directions parallel to the longitudinal axis C.

When the multi-lumen tube 21 is expanded in the directions parallel to the longitudinal axis C, a dimension of the first hole 55A and a dimension of the second hole 57A in the directions parallel to the longitudinal axis C increase. Further, when the dimension of the loop-shaped portion 59 along the linear core axis L decreases, the multi-lumen tube 21 is compressed by the loop-shaped portion 59 between the first hole 55A and the second hole 57A. As a result, a distance between the first hole 55A and the second hole 57A is reduced. It is to be noted that, in the vicinity of the first hole 55B and the second hole 57B, the linear portion 51 is deformed in accordance with the expansion of the multi-lumen tube 21 in the directions parallel to the longitudinal axis C like the vicinity of the first hole 55A and the second hole 57A.

Since the linear portion 51 is deformed as described above, even in the state that the slack parts E1 to E3 are not produced in the linear portion 51 in the endoscope insertion duct 37, the linear portion 51 can be deformed in accordance with the expansion of the multi-lumen tube 21. That is, when the loop-shaped portion 59 is provided to the linear portion 51, the linear portion 51 is deformed in accordance with the expansion of the multi-lumen tube 21 in the directions parallel to the longitudinal axis C without producing the slack parts E1 to E3 in the linear portion 51 in the endoscope insertion duct 37. When the slack parts E1 to E3 are not generated in the linear portion 51 in the endoscope insertion duct 37, the insertability of the insertion section 5 in the endoscope insertion duct 37 is improved.

### (Modifications of First Embodiment)

It is to be noted that each of the first holes 55A and 55B is placed on the proximal direction side with respect to the corresponding second hole 57A or 57B in the first embodiment, but it is not restricted thereto. For example, as shown in FIG. 11, each of the first holes 55A and 55B may be placed to the distal direction side with respect to the corresponding second hole 57A or 57B as a second modification. In this modification, the each first hole 55A or 55B is apart from the corresponding second hole 57A or 57B in the directions parallel to the longitudinal axis C. It is to be noted that a description will be given only as to the configuration near the first hole 55A and the second hole 57A, but the same description can be applied to the configuration near the first hole 55B and the second hole 57B.

As shown in FIG. 11, in the endoscope insertion duct 37, the linear portion 51 is extended toward the proximal direction until it reaches the first hole 55A. Further, in the first hole 55A, the linear portion 51 is extended from the endoscope insertion conduit 37 to the outside of the multi-lumen tube 21. Furthermore, on the outer peripheral portion of the multi-lumen tube 21, the linear portion 51 is extended from the first hole 55A toward the proximal direction until it reaches the second hole 57A. Moreover, in the second hole 57A, the linear portion 51 is inserted into the endoscope insertion duct 37 from the outside of the multi-lumen tube 21. Additionally, in the endoscope insertion duct 37, the linear portion 51 is extended from the second hole 57A toward the distal direction until it reaches the first hole 55A. Further, the linear portion 51 is again extended from the endoscope insertion conduit 37 to the outside of the multi-lumen tube 21 through the first hole 55A. Furthermore, on the outer peripheral portion of the multi-lumen tube 21, the linear portion 51 is extended from the first hole 55A toward the proximal direction until it reaches the second hole 57A. Moreover, the linear portion 51 is again inserted into the endoscope insertion duct 37 from the outside of the multi-lumen tube 21 through the second hole 57A. Additionally, in the endoscope insertion duct 37, the linear portion 51 is extended from the second hole 57A toward the proximal direction. When the linear portion 51 is extended as described above, a loop-shaped portion 61 is formed in the linear portion 51 between the first hole 55A and the second hole 57A.

In the first embodiment and the second modification, the first hole (55A or 55B) and the second hole (57A or 57B) are apart from each other in the directions parallel to the longitudinal axis. Further, the loop-shaped portion (59 or 61) is formed in the linear portion 51 between the first hole (55A or 55B) and the second hole (57A or 57B). When the above-described configuration is adopted, the linear portion 51 is deformed in accordance with the expansion of the multi-lumen tube 21 in the directions parallel to the longitudinal axis C without producing the slack parts (E1 to E3) in the linear portion 51 in the endoscope insertion duct 37.

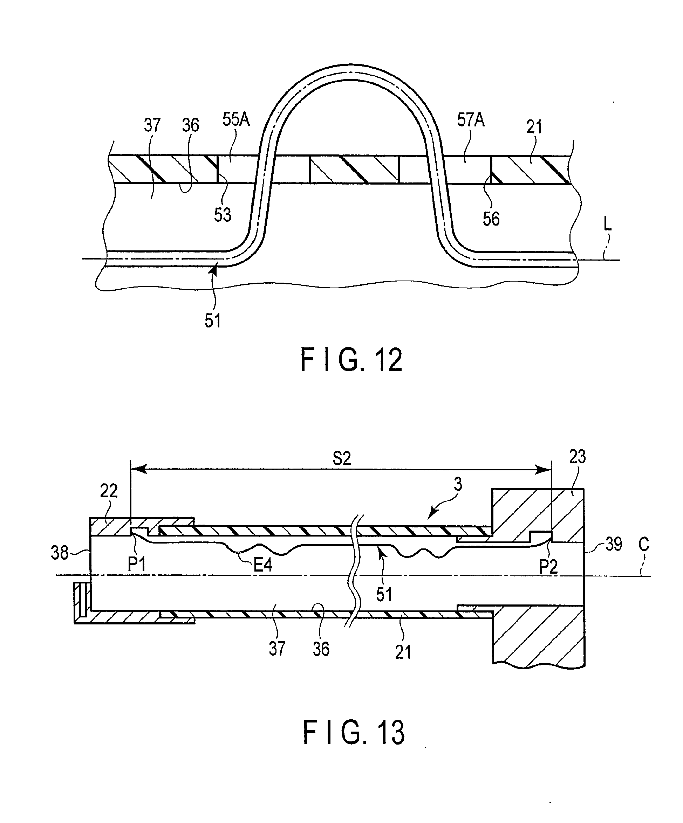

Furthermore, in the first embodiment, although the loop-shaped portion 59 is formed by the linear portion 51 between each of the first holes 55A and 55B and the corresponding second hole 57A or 57B, it is not restricted thereto. For example, as shown in FIG. 12, the loop-shaped portion (59 or 61) may not be formed between the each first hole 55A or 55B and the corresponding second hole 57A or 57B as a third modification. It is to be noted that a description will be given only as to the configuration near the first hole 55A and the second hole 57A, but the same description can be applied to the configuration near the first hole 55B and the second hole 57B.

As shown in FIG. 12, in this modification, the first hole 55A is placed to the distal direction side with respect to the second hole 57A. In the endoscope insertion duct 37, the linear portion 51 is extended toward the proximal direction until it reaches the first hole 55A. Moreover, in the first hole 55A, the linear portion 51 is extended from the endoscope insertion conduit 37 to the outside of the multi-lumen tube 21. Additionally, on the outer peripheral portion of the multi-lumen tube 21, the linear portion 51 is extended from the first hole 55A toward the proximal direction until it reaches the second hole 57A. Further, in the second hole 57A, the linear portion 51 is inserted into the endoscope insertion duct 37 from the outside of the multi-lumen tube 21. Furthermore, in the endoscope insertion duct 37, the linear portion 51 is extended from the second hole 57A toward the proximal direction.

In the first embodiment and the third modification, the first hole (55A or 55B) and the second hole (57A or 57B) different from the first hole (55A or 55B) are formed in the multi-lumen tube 21. Moreover, the linear portion 51 is extended through the endoscope insertion duct 37 between the distal-side connecting position P1 and the proximal-side connecting position P2. Additionally, the linear portion 51 is extended from the endoscope insertion duct 37 to the outside of the multi-lumen tube 21 in the first hole (55A or 55B), and it is inserted into the endoscope insertion duct 37 from the outside of the multi-lumen tube 21 in the second hole (57A or 57B). When the above-described configuration is adopted, in a state that the insertion section 5 moves toward the distal direction in the endoscope insertion duct 37, it is hard for the slack parts (E1 to E3) of the linear portion 51 to be hitched on the insertion section 5.

Further, in the first embodiment, although the first holes 55A and 55B and the second holes 57A and 57B are provided in the multi-lumen tube 21, it is not restricted thereto. For example, as shown in FIG. 13, the first hole (55A or 55B) and the second hole (57A or 57B) may not be provided in the multi-lumen tube 21 as a fourth modification. In this modification, the linear portion 51 is extended from the distal-side connecting position P1 to the proximal-side connecting position P2 in the endoscope insertion duct 37 without passing through the outside of the multi-lumen tube 21. Furthermore, in this modification, like the first embodiment, the linear dimension S1 of the linear portion 51 along the linear core axis L between the distal-side connecting position P1 and the proximal-side connecting position P2 is larger than the parallel axial dimension S2 along the longitudinal axis C between the distal-side connecting position P1 and the proximal-side connecting position P2. Therefore, in the endoscope insertion duct 37, a slack part E4 is produced in the linear portion 51.

In the first embodiment and the fourth modification, the linear dimension S1 of the linear portion 51 along the linear core axis L between the distal-side connecting position P1 and the proximal-side connecting position P2 is larger than the parallel axis dimension S2 along the longitudinal axis C between the distal-side connecting position P1 and the proximal-side connecting position P2. When the above-described configuration is adopted, extensibility of the multi-lumen tube 21 in the directions parallel to the longitudinal axis C can be assured even though the linear portion 51 that does not extend by pulling in the directions parallel to the longitudinal axis C is provided.

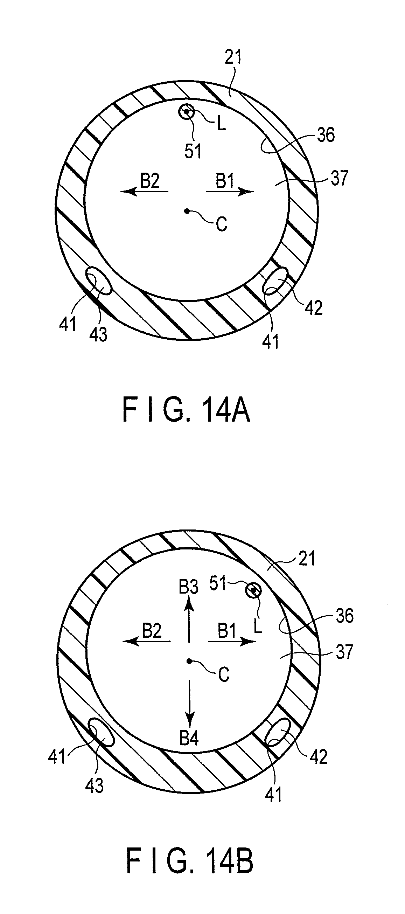

Moreover, in the first embodiment, the bending section 9 can bend in two directions perpendicular to the longitudinal axis C, and the linear portion 51 is placed at substantially the same angular position as one of the bending directions (the direction of the arrow B1 in FIG. 3) of the bending section 9 of the endoscope 2 in the directions around the longitudinal axis, but it is not restricted thereto. For example, as shown in FIG. 14A, the linear portion 51 may be placed to be substantially 90° apart from each of the bending directions (a direction of an arrow B1 and a direction of an arrow B2 in FIG. 14A) of the bending section 9 in the directions around the longitudinal axis as a fifth modification. That is, the linear portion 51 is placed at an angular position deviating from each bending direction of the bending section 9 of the endoscope 2 in the directions around the longitudinal axis. Therefore, an influence of the linear portion 51 on bending of the multi-lumen tube 21 is reduced. Therefore, the multi-lumen tube 21 can readily bend in accordance with the bending section 9, and the bending section 9 can be improved in terms of bendability.

Additionally, as shown in FIG. 14B, the bending section 9 may be capable of bending in four directions perpendicular to the longitudinal axis C as a sixth modification. In this modification, a direction of an arrow B1 and a direction of an arrow B2 in FIG. 14B correspond to first bending directions of the bending section 9, and a direction of an arrow B3 and a direction of an arrow B4 in FIG. 14B correspond to second bending directions of the bending section 9. The each second bending direction is perpendicular to the each first bending direction. In this modification, the linear portion 51 is placed to be substantially 45° apart from one of the first bending directions (the direction of the arrow B1 in FIG. 14B) of the bending section 9 in the directions around the longitudinal axis. Further, the linear portion 51 is placed to be substantially 45° apart from one of the second bending directions (the direction of the arrow B3 in FIG. 14B) of the bending section 9 in the directions around the longitudinal axis. That is, the linear portion 51 is placed at an angular position deviating from the first bending directions and the second bending directions of the bending section 9 of the endoscope 2 in the directions around the longitudinal axis.

In the fifth modification and the sixth modification, the linear portion 51 is placed at an angular position deviating from the bending directions (the first bending direction and the second bending direction) of the bending section 9 of the endoscope 2 in the directions around the longitudinal axis. When the above-described configuration is adopted, an influence of the linear portion 51 on bending of the multi-lumen tube 21 is reduced.

Furthermore, in the first embodiment and the first modification to the sixth modification, the distal-side fixing portion 22 and the proximal-side fixing portion 23 are fixed to the multi-lumen tube 21. Moreover, the distal end of the linear portion 51 is fixed to the distal-side fixing portion 22, and the proximal end of the linear portion 51 is fixed to the proximal-side fixing portion 23. The linear portion 51 has a higher tensile strength in the directions parallel to the longitudinal axis C than the multi-lumen tube 21, and the linear portion 51 is not expanded even when it is pulled in the directions parallel to the longitudinal axis C. As described above, when the linear portion 51 is provided, the tensile strength of the multi-lumen tube 21 in the directions parallel to the longitudinal axis C also increases.

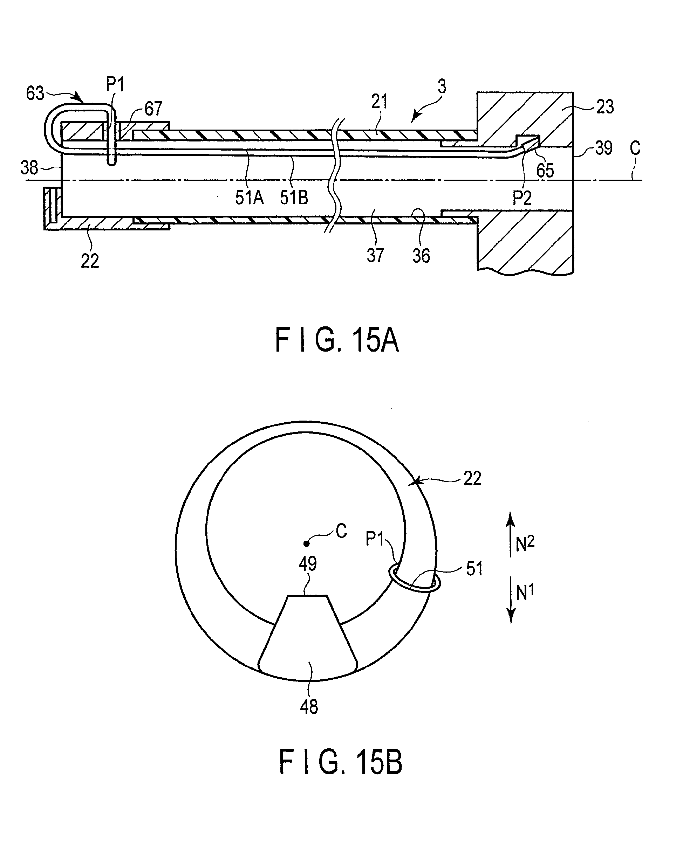

Additionally, in a seventh modification shown in FIG. 15A, two linear portions 51A and 51B are extended between the distal-side connecting position P1 and the proximal-side connecting position P2. The linear portions 51A and 51B are extended through the endoscope insertion duct 37. The linear portions 51A and 51B are formed by an annular member 63. In this modification, the linear portions 51A and 51B have a higher tensile strength in the directions parallel to the longitudinal axis C than the multi-lumen tube 21, and the linear portions 51A and 51B are not stretched even when they are pulled in the directions parallel to the longitudinal axis C.

A caulking member 65 is fixed to one end portion of the annular member 63. Further, the caulking member 65 is fixed to the proximal-side fixing portion 23. As a result, at the proximal-side connecting position P2, proximal ends of the linear portions 51A and 51B are fixed to the proximal-side fixing portion 23.

Furthermore, at the distal-side connecting position P1 of the distal-side fixing portion 22, a through hole 67 that is pierced from the outer peripheral portion of the distal-side fixing portion 22 to the endoscope insertion duct 37 is formed. The other end portion of the annular member 63 is inserted into the endoscope insertion duct 37 via the through hole 67. Moreover, the annular member 63 is extended to an outside of the distal-side fixing portion 22 via the through hole 67 and again inserted into the endoscope insertion duct 37 from the distal-side opening portion 38. Additionally, at a portion of the annular member 63 that is inserted into the endoscope insertion conduit 37 from the through hole 67, the annular member 63 passes through the inner side of a ring. Further, in the endoscope insertion duct 37, the annular member 63 is extended toward the proximal direction. When the annular member 63 is extended as described above, the annular member 63 is attached to the distal-side fixing portion 22. That is, the annular member 63 is attached to the distal-side fixing portion 22 by a Cow hitch. As described above, when the annular member 63 is attached to the distal-side fixing portion 22, distal ends of the linear portions 51A and 51B are fixed to the distal-side fixing portion 22 at the distal-side connecting position P1.

In this modification, the two linear portions 51A and 51B are extended between the distal-side connecting position P1 and the proximal-side connecting position P2. Therefore, the tensile strength of the multi-lumen tube 21 in the directions parallel to the longitudinal axis C further increases. Therefore, it is more difficult for the multi-lumen tube 21 to be expanded by pulling in the directions parallel to the longitudinal axis C, and damage to the endoscope cleaning sheath 3 due to pulling in the directions parallel to the longitudinal axis C is further effectively avoided.

Further, in the first embodiment, although the distal-side connecting position P1 of the linear portion 51 is placed at the angular position that is substantially 180° apart from the nozzle 48 in the directions around the longitudinal axis, it is not restricted thereto. For example, as shown in FIG. 15B, the distal-side connecting position P1 of the linear portion 51 may be placed at an angular position that is substantially 60° apart from one in the directions around the longitudinal axis as an eighth modification.

Here, a given one direction perpendicular to the longitudinal axis C is determined as a first perpendicular direction (a direction of an arrow N1 in FIG. 15B), and an opposite direction to the first perpendicular direction is determined as a second perpendicular direction (a direction of an arrow N2 in FIG. 15B). In this modification, a shape is different from the distal-side fixing portion 22 in the first embodiment. That is, in this modification, the nozzle 48 as the fluid emitting portion protrudes toward the second perpendicular direction. The nozzle 48 emits air and water (a fluid) from the first perpendicular direction toward the second perpendicular direction. An emission port 49 is formed at a second-perpendicular-direction-side end of the nozzle 48. The nozzle 48 emits the fluid from the emission port 49 toward the observation window 15. The distal-side connecting position P1 of the linear portion 51 is placed to the second perpendicular direction side with respect to the emission port 49 of the nozzle 48. It is to be noted that, in this modification, the linear portion 51 is fixed to the distal-side fixing portion 22 at the distal-side connecting position P1 by the Cow hitch like the seventh modification.

A wall thickness in the distal-side fixing portion 22 increases as a position in it gets closer to the nozzle 48 in the directions around the longitudinal axis. Therefore, in this modification, in which the distal-side connecting position P1 of the linear portion 51 is placed at an angular position that is substantially 60° apart from the nozzle 48 in the directions around the longitudinal axis, the distal end of the linear portion 51 is fixed at the distal-side fixing portion 22 in a region having a large wall thickness as compared with the foregoing embodiment and modifications. When the multi-lumen tube 21 is pulled in the directions parallel to the longitudinal axis C, a force generated by pulling acts on the linear portion 51. Therefore, when the multi-lumen tube 21 is pulled, at the distal-side connecting position P1 of the distal-side fixing portion 22, the force acts on the distal-side fixing portion 22 from the linear portion 51. In this modification, since the distal end of the linear portion 51 is fixed to the distal-side fixing portion 22 in the region having the large wall thickness, damage to the distal-side fixing portion 22 due to the force from the linear portion 51 is effectively avoided.

The air or the water is emitted from the nozzle 48 in a state that the first perpendicular direction is a vertically downward direction. Therefore, the water emitted from the nozzle 48 toward the second perpendicular direction that is a vertically upward direction moves toward the first perpendicular direction by gravity and returns to a position near the emission port 49. In this modification, at the distal-side connecting position P1 located on the second perpendicular direction side with respect to the emission port 49, the linear portion 51 that can readily collect water is fixed to the distal-side fixing portion 22. That is, the linear portion 51 that can easily collect water is placed at a position apart from the emission port 49 of the nozzle 48. Therefore, a large amount of water emitted from the emission port 49 can be prevented from collecting near the emission port 49. Since a large amount of water is not stored near the emission port 49, the collected water can be effectively prevented from being blown up toward the second perpendicular direction by the emitted air when the air is emitted from the emission port 49.

### (Second Embodiment)

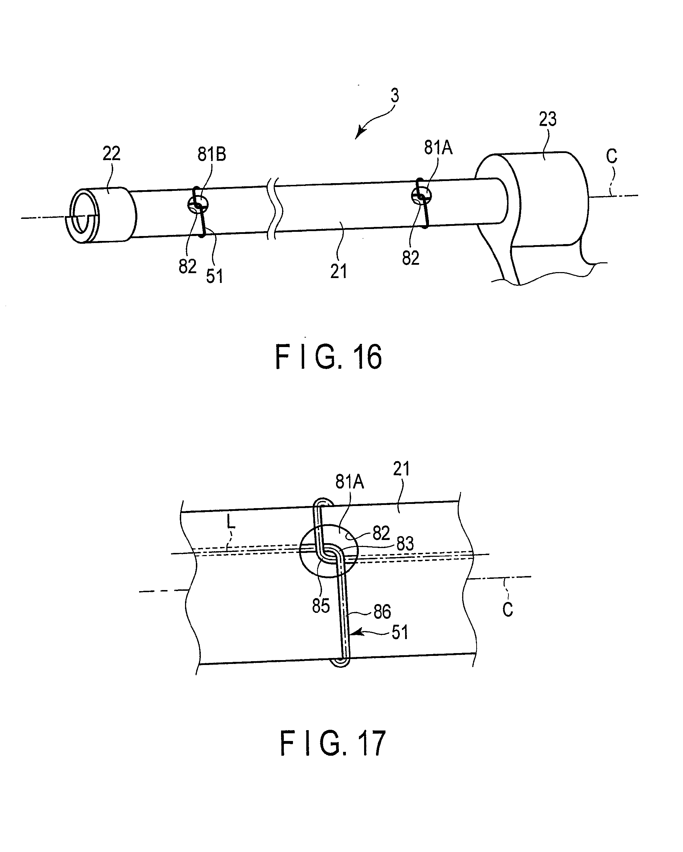

A second embodiment according to the present invention will now be described with reference to FIG. 16 to FIG. 19B. A second embodiment is provided by modifying the configuration of the first embodiment as follows. It is to be noted that like reference numerals denote parts equal to those in the first embodiment, thereby omitting a description thereof.

FIG. 16 is a view showing an endoscope cleaning sheath 3 according to this embodiment. As shown in FIG. 16, in this embodiment, likewise, a multi-lumen tube 21, a distal-side fixing portion 22, and a proximal-side fixing portion 23 are provided to the endoscope cleaning sheath 3. In this embodiment, holes 81A and 81B are defined in the multi-lumen tube 21 as a sheath main body by hole defining portions 82. The hole 81A is placed to the proximal direction side with respect to the hole 81B.

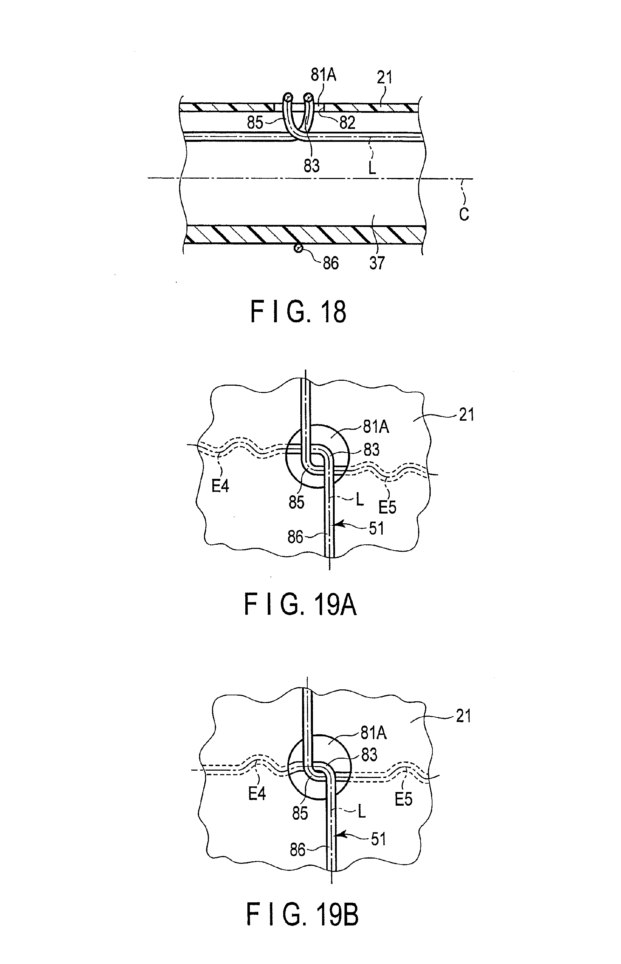

FIG. 17 and FIG. 18 are views showing configurations of the multi-lumen tube 21 and a linear portion 51 near a given one 81A of holes. It is to be noted that a description will be given as to a configuration near the hole 81A alone, but the same description can be applied to a configuration near the hole 81B. Further, in this embodiment, although the two holes 81A and 81B are provided, defining one or more holes (81A and 81B) in the multi-lumen tube 21 can suffice. As shown in FIG. 17 and FIG. 18, an endoscope insertion duct 37 communicates with the outside of the multi-lumen tube 21 through the hole 81A.

In this embodiment, like the first embodiment, the linear portion 51 is extended along a linear core axis L through an endoscope insertion conduit 37 between a distal-side connecting position P1 and a proximal-side connecting position P2. In the endoscope insertion duct 37, the linear portion 51 is extended toward the proximal direction until it reaches the hole 81A. An external extended portion 83, which is extended from the endoscope insertion duct 37 to the outside of the multi-lumen tube 21 in the hole 81A (81B), is provided to the linear portion 51. Further, a duct inserting portion 85, which is inserted into the endoscope insertion duct 37 from the outside of the multi-lumen tube 21 in the hole 81A (81B), is provided to the linear portion 51. The linear portion 51 is extended toward one of circumferential directions of the multi-lumen tube 21 (directions around the longitudinal direction) between the external extended portion 83 and the duct inserting portion 85. That is, a circumferential extended portion 86, which is extended along the circumferential directions of the multi-lumen tube 21 between the external extended portion 83 and the duct inserting portion 85, is provided to the linear portion 51. The circumferential extended portion 86 is extended to make a circuit of an outer peripheral portion of the multi-lumen tube 21 from the hole 81A to the hole 81A. The linear portion 51 inserted into the endoscope insertion duct 37 from the hole 81A is extended from the hole 81A toward the proximal direction.

In this embodiment, when the linear portion 51 is provided, the tensile strength of the multi-lumen tube 21 in directions parallel to a longitudinal axis C increases. Therefore, it is difficult to expand the multi-lumen tube 21 by pulling in the directions parallel to the longitudinal axis C, and damage to the endoscope cleaning sheath 3 due to pulling in the directions parallel to the longitudinal axis C is effectively avoided.

Furthermore, since the linear portion 51 is extended as described above, like the first embodiment, a linear dimension S1 of the linear portion 51 along the linear core axis L between a distal-side connecting position P1 and a proximal-side connecting position P2 is larger than a parallel axial dimension S2 along the longitudinal axis C between the distal-side connecting position P1 and the proximal-side connecting position P2. Therefore, slack parts are produced in the linear portion 51. Therefore, even when the linear portion 51 that is not extended by pulling in the directions parallel to the longitudinal axis C is provided, bendability, etc. of a bending section 9 can be assured.

Moreover, in the linear portion 51 according to this embodiment, the external extended portion 83 is extended to the outside of the multi-lumen tube 21 in the hole 81A (81B). Additionally, in the linear portion 51, the circumferential extended portion 86 is extended along the circumferential directions on the outer peripheral portion of the multi-lumen tube 21, and the duct inserting portion 85 is inserted into the endoscope insertion duct 37 from the hole 81A (81B). Since the linear portion 51 is extended as described above, when the insertion section 5 moves in the endoscope insertion duct 37 toward the distal direction, the slack parts located to the proximal direction side with respect to the hole 81A (81B) do not move to the distal direction side from the hole 81A. As a result, like the first embodiment, when the insertion section 5 moves in the endoscope insertion duct 37 toward the distal direction, it is hard for the slack parts of the linear portion 51 to be hitched on the insertion section 5. Therefore, even when the slack parts are generated in the linear portion 51, insertability of the insertion section 5 in the endoscope insertion duct 37 can be assured.

Further, in this embodiment, since the linear portion 51 is extended as described above in a part near the hole 81A (81B), even when the multi-lumen tube 21 is pulled in the directions parallel to the longitudinal axis C, a large force does not act on the multi-lumen tube 21 from the linear portion 51 in the hole 81A (81B). Therefore, damage to the multi-lumen tube 21 due to a force acting on the multi-lumen tube 21 from the linear portion 51 can be effectively avoided.

FIG. 19A and FIG. 19B are views each showing an extended state of the linear portion 51 near a given one 81A of the holes. FIG. 19A shows a state that the endoscope cleaning sheath 3 is attached to the endoscope 2, and FIG. 19B is a view showing a state that the insertion section 5 moves in the endoscope insertion duct 37 toward the proximal direction in removal of the endoscope cleaning sheath 3 from the endoscope 2. As shown in FIG. 19A and FIG. 19B, the external extended portion 83 is hitched on the duct inserting portion 85 in the hole 81A (81B). As shown in FIG. 19A, in a state that the endoscope cleaning sheath 3 is attached to the endoscope 2, like the first embodiment, slack parts E4 and E5 of the linear portion 51 are dispersed over the entire length of the endoscope insertion duct 37 in the directions parallel to the longitudinal axis C. Here, the slack part E4 is a slack part that is produced between the hole 81A and the hole 81B, and the slack part E5 is a slack part that is produced on the proximal end direction side with respect to the hole 81A.

As shown in FIG. 19B, in removal of the endoscope cleaning sheath 3 from the endoscope 2, when the insertion section 5 moves in the endoscope insertion duct 37 toward the proximal direction, the slack parts E4 and E5 are pushed by the insertion section 5 toward the proximal direction in the endoscope insertion duct 37. As a result, the slack parts E4 and E5 move toward the proximal direction. In the linear portion 51 according to this embodiment, the external extended portion 83 is hitched on the duct inserting portion 85 in the hole 81A. Therefore, when the slack part E5 located to the proximal direction side with respect to the hole 81A moves toward the proximal direction, the duct inserting portion 85 abuts on the external extended portion 83 in the hole 81A, and the external extended portion 83 and the duct inserting portion 85 push each other in the hole 81A. When the external extended portion 83 and the duct inserting portion 85 push each other in the hole 81A, the movement of the slack part E4 (i.e., the slack part E4 located to the distal direction side with respect to the hole 81A) located between the hole 81A and the hole 81B toward the proximal direction side from the hole 81A is effectively avoided. When the movement of the slack part E4 toward the proximal direction side from the hole 81A is avoided, crowding of the slack parts in a region to the proximal direction side with respect to the hole 81A can be effectively avoided after removing the insertion section 5 from the endoscope insertion duct 37.

As described above, in this embodiment, the slack parts E4 and E5 of the linear portion 51 are not congested in a proximal-direction-side region of the endoscope insertion duct 37 by removal of the endoscope cleaning sheath 3 from the endoscope 2. Therefore, even after repeating the attachment and the removal of the endoscope cleaning sheath 3 with respect to the endoscope 2, the slack parts E4 and E5 of the linear portion 51 are not congested in the proximal-direction-side region of the endoscope insertion duct 37. As a result, even after repeating the attachment and the removal of the endoscope cleaning sheath 3 with respect to the endoscope 2, the insertability of the insertion section 5 in the endoscope insertion duct 37 can be assured.

### (Modifications of First Embodiment and Second Embodiment)

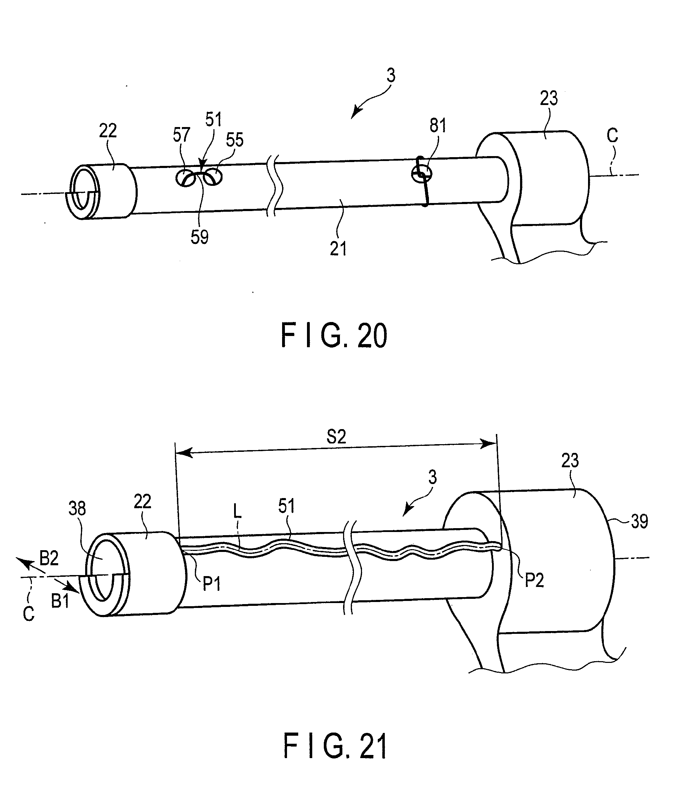

It is to be noted that, as a ninth modification, the extended state of the linear portion 51 in the first embodiment may be combined with the extended state of the linear portion 51 in the second embodiment as shown in FIG. 20. In this modification, in the multi-lumen tube 21, a first hole 55 having the same configuration as those of the first holes 55A and 55B in the first embodiment, a second hole 57 having the same configuration as those of the second holes 57A and 57B in the first embodiment, and a third hole 81 having the same configuration as those of the holes 81A and 81B in the second embodiment are provided.

In this modification, the first hole 55 is placed to the proximal direction side with respect to the second hole 57. Further, the third hole 81 is placed to the proximal direction side with respect to the first hole 55 and the second hole 57. In the vicinity of the first hole 55 and the second hole 57, the linear portion 51 is extended like the extended state near the first hole 55A and the second hole 57A in the first embodiment. That is, in the endoscope insertion duct 37, the linear portion 51 is extended toward the proximal direction until it reaches the first hole 55. Furthermore, in the first hole 55, the linear portion 51 is extended from the endoscope insertion conduit 37 to the outside of the multi-lumen tube 21. Moreover, on the outer peripheral portion of the multi-lumen tube 21, the linear portion 51 is extended toward the distal direction from the first hole 55 to the second hole 57. Additionally, in the second hole 57, the linear portion 51 is inserted into the endoscope insertion duct 37 from the outside of the multi-lumen tube 21. Further, in the endoscope insertion duct 37, the linear portion 51 is extended from the second hole 57 toward the proximal direction. Therefore, the loop-shaped portion 59 is formed by the linear portion 51 between the first hole 55 and the second hole 57.

Furthermore, in this modification, in the vicinity of the third hole 81, the linear portion 51 is extended like the extended state near the hole 81A in the second embodiment That is, in the linear portion 51, the external extended portion 83 is extended from the endoscope insertion duct 37 to the outside of the multi-lumen tube 21 in the third hole 81, and the duct inserting portion 85 is inserted into the endoscope insertion duct 37 from the outside of the multi-lumen tube 21 in the third hole 81. Moreover, the circumferential extended portion 86 of the linear portion 51 is extended along the circumferential directions of the multi-lumen tube 21 (the directions around the longitudinal axis) between the external extended portion 83 and the duct inserting portion 85. In this modification, like the first embodiment and the second embodiment, the insertability of the insertion section 5 in the endoscope insertion duct 37 can be assured.

### (Third Modification)

A third embodiment according to the present invention will now be described with reference to FIG. 21. The third embodiment is provided by changing the configuration of the first embodiment as follows. It is to be noted that like reference numerals denote parts equal to those in the first embodiment to omit a description thereof.

FIG. 21 is a view showing a configuration of an endoscope cleaning sheath 3 according to this embodiment. As shown in FIG. 21, in this embodiment, likewise, a multi-lumen tube 21 as a sheath main body, a distal-side fixing portion 22, and a proximal-side fixing portion 23 are provided to the endoscope cleaning sheath 3. Additionally, a linear portion 51 has a distal end fixed to the distal-side fixing portion 22 at a distal-side connecting position P1 and a proximal end fixed to the proximal-side fixing portion 23 at a proximal-side connecting position P2. However, in this embodiment, the linear portion 51 is extended on an outer peripheral portion of the multi-lumen tube 21 from the distal-side connecting position P1 to the proximal-side connecting position P2 without passing through the endoscope insertion duct 37.

In this embodiment, like the first embodiment, a linear dimension S1 of the linear portion 51 along a linear core axis L between the distal-side connecting position P1 and the proximal-side connecting position P2 is larger than a parallel axial dimension S2 along a longitudinal axis C between the distal-side connecting position P1 and the proximal-side connecting position P2. Further, the linear portion 51 is placed at an angular position deviating from bending directions (a direction of an arrow B1 and a direction of an arrow B2 in FIG. 21) of a bending section 9 of an endoscope 2 in directions around the longitudinal axis.

In this embodiment, likewise, when the linear portion 51 is provided, the tensile strength of the multi-lumen tube 21 in directions parallel to the longitudinal axis C increases. Therefore, it is hard to expand the multi-lumen tube 21 by pulling in the directions parallel to the longitudinal axis C, and damage to the endoscope cleaning sheath 3 due to pulling in the directions parallel to the longitudinal axis C can be effectively avoided.

Furthermore, in this embodiment, the linear portion 51 is extended from the distal-side connecting position P1 to the proximal-side connecting position P2 on the outer peripheral portion of the multi-lumen tube 21 without passing through the endoscope insertion duct 37. Therefore, even when the linear portion 51 is provided, the insertability of the insertion section 5 in the endoscope insertion duct 37 can be assured.

### (Modification of Third Embodiment)

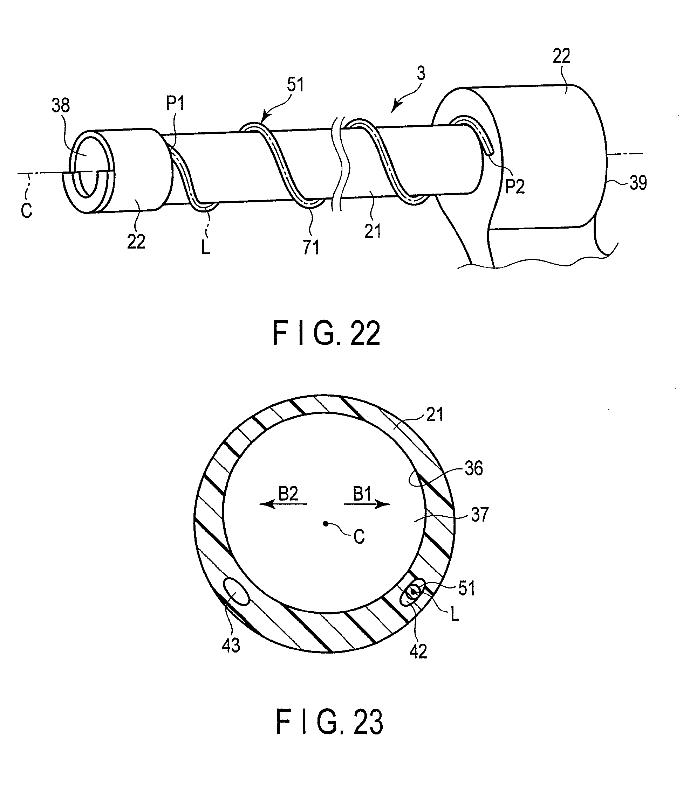

It is to be noted that, as a 10th modification, the linear portion 51 may be spirally extended on the outer peripheral portion of the multi-lumen tube 21 in the directions parallel to the longitudinal axis C as shown in FIG. 22. That is, the linear portion 51 includes a spiral portion 71 spirally extended in the directions parallel to the longitudinal axis C. In this modification, the like third embodiment, the linear portion 51 is extended from the distal-side connecting position P1 to the proximal-side connecting position P2 on the outer peripheral portion of the multi-lumen tube 21 without passing through the endoscope insertion duct 37.

In this modification, since the linear portion 51 is spirally extended, in a state that the multi-lumen tube 21 is bent in accordance with a bending motion of the bending section 9, the linear portion 51 can readily follow the bending motion of the bending section 9. Therefore, the bendability of the bending section 9 is further improved.

### (Fourth Embodiment)

A third embodiment according to the present invention will now be described with reference to FIG. 23. The fourth embodiment is provided by modifying the configuration of the first embodiment as follows. It is to be noted that like reference numerals denote parts equal to those in the first embodiment to omit a description thereof.

FIG. 23 is a view showing an internal configuration of a multi-lumen tube 21 of an endoscope cleaning sheath 3 according to this embodiment. In this embodiment, likewise, a multi-lumen tube 21 as a sheath main body, a distal-side fixing portion 22, and a proximal-side fixing portion 23 are provided to the endoscope cleaning sheath 3. Additionally, a linear portion 51 has a distal end fixed to the distal-side fixing portion 22 at a distal-side connecting position P1 and a proximal end fixed to the proximal-side fixing portion 23 at a proximal-side connecting position P2. Further, as shown in FIG. 23, in the multi-lumen tube 21, an endoscope insertion duct 37, an air supply duct 42, and a water supply duct 43 are defined. Furthermore, the air supply duct 42 and the water supply duct 43 as fluid ducts are separated from the endoscope insertion conduit 37. However, in this embodiment, the linear portion 51 is extended in the air supply conduit 42 from the distal-side connecting position P1 and the proximal-side connecting position P2 without passing through the endoscope insertion duct 37.

In this embodiment, like the first embodiment, a linear dimension S1 of the linear portion 51 along a linear core axis L between the distal-side connecting position P1 and the proximal-side connecting position P2 is larger than a parallel axial dimension S2 along a longitudinal axis C between the distal-side connecting position P1 and the proximal-side connecting position P2. Further, the linear portion 51 is placed at an angular position deviating from bending directions (a direction of an arrow B1 and a direction of an arrow B2 in FIG. 22) of a bending section 9 of an endoscope 2 in directions around the longitudinal axis.

In this embodiment, likewise, when the linear portion 51 is provided, the tensile strength of the multi-lumen tube 21 in directions parallel to the longitudinal axis C increases. Therefore, the multi-lumen tube 21 is hard to be expanded by pulling in the directions parallel to the longitudinal axis C, and damage to the endoscope cleaning sheath 3 due to pulling in the directions parallel to the longitudinal axis C can be effectively avoided.

Furthermore, in this embodiment, the linear portion 51 is extended from the distal-side connecting position P1 to the proximal-side connecting position P2 in the air supply duct 42 without passing through the endoscope insertion duct 37. Therefore, even when the linear portion 51 is provided, the insertability of the insertion section 5 in the endoscope insertion duct 37 can be assured.

### (Modification of Fourth Embodiment)

It is to be noted that the linear portion 51 is extended in the air supply duct 42 in the fourth embodiment, but the linear portion 51 may be extended from the distal-side connecting position P1 to the proximal-side connecting position P2 in the water supply duct 43 in one modification. Moreover, in another modification, two linear portions 51 may be provided, one linear portion 51 may be extended in the air supply duct 42, and the other linear portion 51 may be extended in the water supply duct 43.

In the third embodiment and its modification, each fluid duct (42 or 43) separated from the endoscope insertion duct 37 is defined. Additionally, the linear portion 51 is extended from the distal-side connecting position P1 to the proximal-side connecting position P2 in the fluid duct (42 or 43). As a result, even when the linear portion 51 is provided, the insertability of the insertion section 5 in the endoscope insertion duct 37 can be assured.

### (Fifth Embodiment)

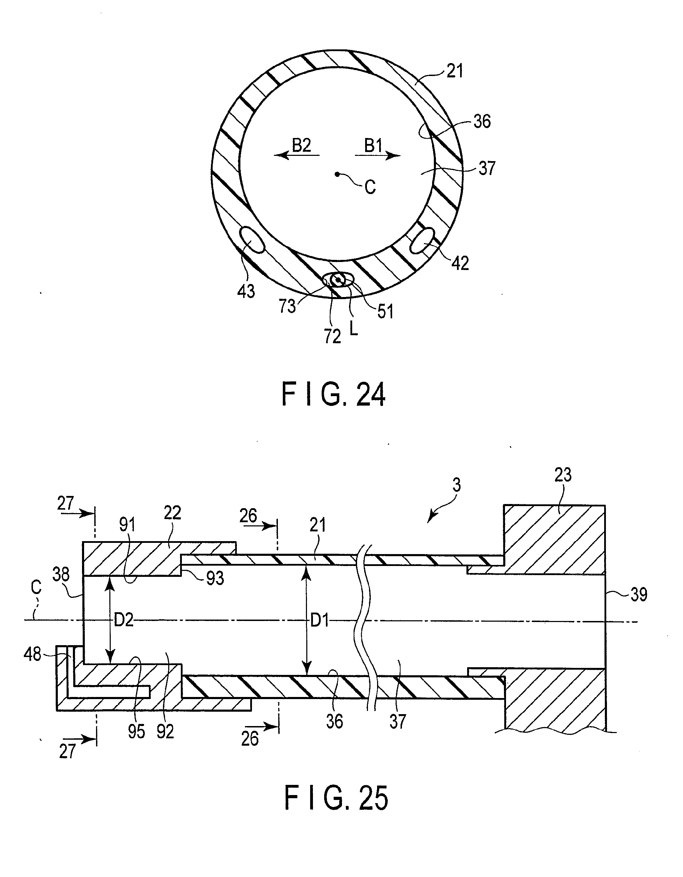

A fifth embodiment according to the present invention will now be described with reference to FIG. 24. The fifth embodiment is provided by modifying the configuration of the first embodiment as follows. It is to be noted that like reference numerals denote parts equal to those in the first embodiment to omit a description thereof.

FIG. 24 is a view showing an internal configuration of a multi-lumen tube 21 of an endoscope cleaning sheath 3 according to this embodiment. In this embodiment, likewise, a multi-lumen tube 21 as a sheath main body, a distal-side fixing portion 22, and a proximal-side fixing portion 23 are provided to the endoscope cleaning sheath 3. Additionally, a linear portion 51 has a distal end fixed to the distal-side fixing portion 22 at a distal-side connecting position P1 and a proximal end fixed to the proximal-side fixing portion 23 at a proximal-side connecting position P2. Further, as shown in FIG. 24, in the multi-lumen tube 21, an endoscope insertion duct 37, an air supply duct 42, and a water supply duct 43 are defined. Furthermore, the air supply duct 42 and the water supply duct 43 as fluid ducts are separated from the endoscope insertion conduit 37. However, in this embodiment, a separation duct 72 is defined by a separation duct defining portion 73 in the multi-lumen tube 21 as the sheath main body in a state that the separation duct 72 is separated from the endoscope insertion duct 37 and fluid ducts (the air supply conduit 42 and the water supply conduit 43). Further, the linear portion 51 is extended in the separation conduit 72 from the distal-side connecting position P1 to the proximal-side connecting position P2 without passing through the endoscope insertion duct 37.

In this embodiment, like the first embodiment, a linear dimension S1 of the linear portion 51 along a linear core axis L between the distal-side connecting position P1 and the proximal-side connecting position P2 is larger than a parallel axial dimension S2 along a longitudinal axis C between the distal-side connecting position P1 and the proximal-side connecting position P2. Further, the linear portion 51 is placed at an angular position deviating from bending directions (a direction of an arrow B1 and a direction of an arrow B2 in FIG. 24) of a bending section 9 of an endoscope 2 in directions around the longitudinal axis.

In this embodiment, likewise, when the linear portion 51 is provided, the tensile strength of the multi-lumen tube 21 in directions parallel to the longitudinal axis C increases. Therefore, the multi-lumen tube 21 is hard to be stretched by pulling in the directions parallel to the longitudinal axis C, and damage to the endoscope cleaning sheath 3 due to pulling in the directions parallel to the longitudinal axis C can be effectively avoided.

Furthermore, in this embodiment, the linear portion 51 is extended in the separation duct 72 from the distal-side connecting position P1 to the proximal-side connecting position P2 without passing through the endoscope insertion duct 37. Therefore, even when the linear portion 51 is provided, the insertability of the insertion section 5 in the endoscope insertion duct 37 can be assured.

### (Sixth Embodiment)

A sixth embodiment according to the present invention will now be described with reference to FIG. 25 to FIG. 28. The sixth embodiment is provided by modifying the configuration of the first embodiment as follows. It is to be noted that like reference numerals denote parts equal to those in the first embodiment to omit a description thereof.

FIG. 25 is a view showing a configuration of an endoscope cleaning sheath 3 according to this embodiment. In this embodiment, likewise, a multi-lumen tube 21 as a sheath main body, a distal-side fixing portion 22, and a proximal-side fixing portion 23 are provided to the endoscope cleaning sheath 3. Further, in the multi-lumen tube 21, an endoscope insertion duct 37 is defined by an insertion duct defining portion 36 along a longitudinal axis C. It is to be noted that a linear portion 51 is not provided in this embodiment.

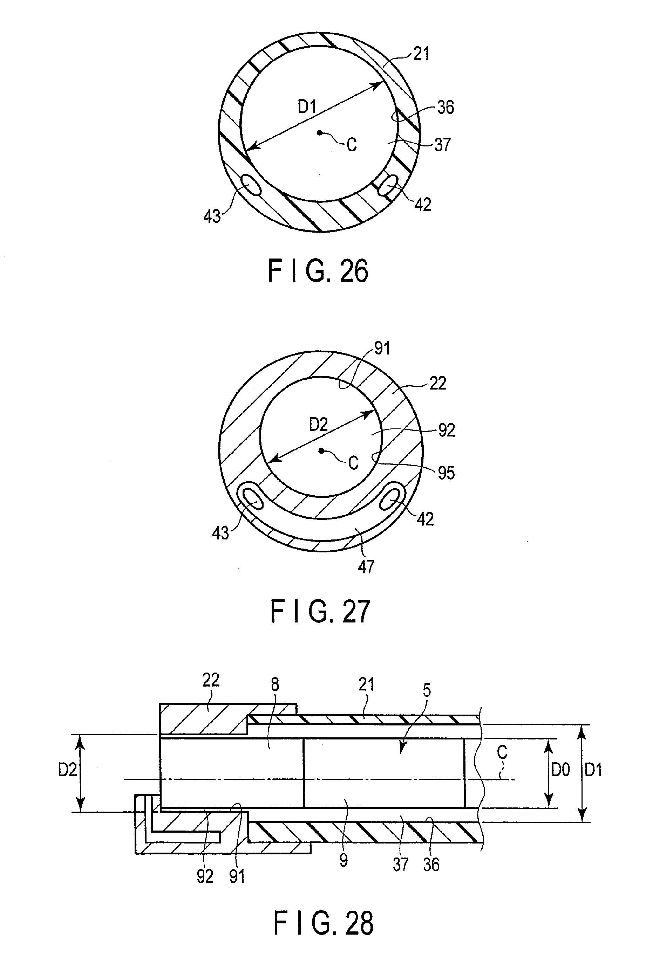

FIG. 26 is a cross-sectional view taken along a line 26-26 in FIG. 25. As shown in FIG. 26, in this embodiment, like the first embodiment, an air supply duct 42 and a water supply duct 43 are defined by fluid duct defining portions 41. Moreover, the multi-lumen tube 21 is made of silicone by extrusion molding, and it has flexibility. Since the multi-lumen tube 21 is formed by the extrusion molding, in the multi-lumen tube 21, as shown in FIG. 25, a cross section perpendicular to the longitudinal axis C has the same shape over the entire length in directions parallel to the longitudinal axis C. Therefore, a cross section of the endoscope insertion duct 37 perpendicular to the longitudinal axis C has the same shape over the entire length in the directions parallel to the longitudinal axis C. That is, the insertion duct defining portion 36 defines a diameter dimension of the endoscope insertion duct 37 as a first diameter dimension D1 over the entire length in the directions parallel to the longitudinal axis C.

FIG. 27 is a cross-sectional view taken along a line 27-27 in FIG. 25. As shown in FIG. 25 and FIG. 27, in this embodiment, like the first embodiment, a merging portion 47 and a nozzle 48 as a fluid emitting portion are provided in the distal-side fixing portion 22. The distal-side fixing portion 22 is made of a material such as polysulfone that is harder than the multi-lumen tube 21.

In this embodiment, an intermediary duct defining portion 91 defines an intermediary duct 92 in the distal-side fixing portion 22. A proximal end of the intermediary duct 92 communicates with a distal end of the endoscope insertion duct 37. In the distal-side fixing portion 22, a distal-side opening portion 38 is provided. A distal end of the intermediary conduit 92 is opened at the distal-side opening portion 38 with respect to an outside of the distal-side fixing portion 22. Therefore, the distal-side opening portion 38 communicates with the endoscope insertion duct 37 through the intermediary duct 92.

The intermediary duct defining portion 91 defines a diameter dimension of the intermediary duct 92 as a second diameter dimension D2 over the entire length in the directions parallel to the longitudinal axis C. The second diameter dimension D2 is smaller than the first diameter dimension D1 of the endoscope insertion duct 37. Therefore, a step portion 93 is formed between the insertion duct defining portion 36 and the intermediary duct defining portion 91. FIG. 28 is a view showing configurations of a distal portion of the endoscope 2 and a distal portion of the endoscope cleaning sheath 3 in a state that the endoscope cleaning sheath 3 is attached to the endoscope 2 according to this embodiment. As shown in FIG. 28, the second diameter dimension D2 of the intermediary duct 92 is larger than an outer diameter dimension D0 of the insertion section 5. However, a difference between the second dimension D2 and the outer diameter dimension D0 of the insertion section 5 is 0.1 mm or less. Therefore, in the state that the endoscope cleaning sheath 3 is attached to the endoscope 2, there is almost no gap between the outer peripheral portion of the insertion section 5 and the intermediary duct defining portion 91.

Further, in a state that the endoscope cleaning sheath 3 is attached to the endoscope 2, a bending section 9 of the insertion section 5 is placed to the proximal direction side with respect to a proximal end of the intermediary duct 92 (i.e., a proximal end of the intermediary duct defining portion 91). Therefore, in the state that the endoscope cleaning sheath 3 is attached to the endoscope 2, the bending section 9 is placed in the endoscope insertion duct 37 provided inside the multi-lumen tube 21 having flexibility. Furthermore, the intermediary duct defining portion 91 of the distal-side fixing portion 22 is thinly coated with a water-shedding coating 95 such as a silicone oil or a silicone coat.

In the endoscope cleaning sheath according to each of Patent Literature 1 and Patent Literature 2, a distal-side opening portion is provided on a distal surface of a multi-lumen tube as a sheath main body. Moreover, a distal end of an endoscope insertion duct is directly opened at the distal-side opening portion with respect to the outside of the distal-side fixing portion. In such a configuration, in a state that the endoscope cleaning sheath is attached to the endoscope, water is apt to flow from the distal-side opening portion into a gap between an insertion duct defining portion that defines the endoscope insertion duct and an outer peripheral portion of an insertion section. Therefore, water easily collects in the gap between the insertion duct defining portion and the outer peripheral portion of the insertion section. If observation is performed through an observation window in a state that water has collected in the gap between the insertion duct defining portion and the outer peripheral portion of the insertion section, the water might possibly flow to the distal surface of the insertion section from the gap between the insertion duct defining portion and the outer peripheral portion of the insertion section. When the water flows out to the distal surface of the insertion section, the water adheres to the observation window, and an observation performance of a subject is lowered.

Reducing a diameter dimension of the endoscope insertion duct in the multi-lumen tube enables the gap between the insertion duct defining portion and the outer peripheral portion of the insertion section to be decreased. However, since the multi-lumen tube as the sheath main body is formed by the extrusion molding, a cross section perpendicular to the longitudinal axis has the same shape over the entire length in the directions parallel to the longitudinal axis. Therefore, in case of reducing the diameter dimension of the endoscope insertion duct, the diameter dimension of the endoscope insertion duct must be reduced over the entire length in the directions parallel to the longitudinal axis. Additionally, in general, the multi-lumen tube is made of a material having flexibility. In a case that the endoscope insertion duct having a small diameter dimension over the entire length is formed into the multi-lumen tube, frictional resistance increases between the outer peripheral portion of the insertion section and the insertion duct defining portion during movement of the insertion section in the endoscope insertion duct. Therefore, insertability of the insertion section into the endoscope insertion duct and removability of the insertion section from the endoscope insertion duct are lowered. That is, movability of the insertion section in the endoscope insertion duct is decreased.

Thus, in this embodiment, as will be described later, there are provided the endoscope cleaning sheath 3 and the endoscope apparatus 1 in which water hardly collects in a gap between the insertion duct defining portion 36 and the outer peripheral portion of the insertion section 5, and in which the mobility of the insertion section 5 in the endoscope insertion duct 37 is assured. That is, in this embodiment, the intermediary duct 92 having a proximal end communicating with the distal end of the endoscope insertion duct 37 is defined in the distal-side fixing portion 22. Further, a distal end of the intermediary conduit 92 is opened with respect to the outside of the distal-side fixing portion 22. The intermediary duct 92 has the second diameter dimension smaller than the first diameter dimension D1 of the endoscope insertion duct 37 over the entire length in the directions parallel to the longitudinal axis C. Since the second radial dimension D2 of the intermediary duct 92 is small, in the state that the endoscope cleaning sheath 3 is attached to the endoscope 2, a gap between the outer peripheral portion of the insertion section 5 and the intermediary duct defining portion 91 is small. Therefore, water hardly passes through the gap between the outer peripheral portion of the insertion section 5 and the intermediary duct defining portion 91, and the water can be effectively prevented from flowing into the gap between the insertion duct defining portion 36 and the outer peripheral portion of the insertion section 5 from the distal-side opening portion 38. Therefore, it is difficult for water to collect in the gap between the insertion duct defining portion 36 and the outer peripheral portion of the insertion section 5.

Furthermore, in this embodiment, a difference between the outer diameter dimension D0 of the insertion section 5 and the second diameter dimension D2 of the same is 0.1 mm or less. Therefore, it is hard for the water to pass through the gap between the outer peripheral portion of the insertion section 5 and the intermediary duct defining portion 91, and the water can be further effectively prevented from flowing into the gap between the insertion duct defining portion 36 and the outer peripheral portion of the insertion section 5 from the distal-side opening portion 38.

Moreover, in this embodiment, the intermediary duct defining portion 91 is coated with the water-shedding coating 95. Therefore, it is harder for the water to pass through the gap between the outer peripheral portion of the insertion section 5 and the intermediary duct defining portion 91, and the water can be further effectively prevented from flowing into the gap between the insertion duct defining portion 36 and the outer peripheral portion of the insertion section 5 from the distal-side opening portion 38.

As described above, in this embodiment, since the water is prevented from flowing into the gap between the insertion duct defining portion 36 and the outer peripheral portion of the insertion section 5 from the distal-side opening portion 38, it is difficult for water to collect in the gap between the insertion duct defining portion 36 and the outer peripheral portion of the insertion section 5. Therefore, in the case of performing observation through the observation window, the water is prevented from adhering to the observation window, and an observation performance of a subject can be assured.

Further, in the endoscope insertion duct 37 provided in the multi-lumen tube 21 having flexibility, a diameter dimension is the first diameter dimension D1 larger than the second diameter dimension D2 over the entire length in the directions parallel to the longitudinal axis C. Therefore, during movement of the insertion section 5 in the endoscope insertion duct 37, frictional resistance does not increase between the outer peripheral portion of the insertion section 5 and the insertion duct defining portion 36. Therefore, insertability of the insertion section 5 into the endoscope insertion duct 37 and removability of the insertion section 5 from the endoscope insertion duct 37 can be assured. That is, movability of the insertion section 5 in the endoscope insertion duct 37 can be assured.

Furthermore, in the intermediary duct 62 provided in the distal-side fixing portion 22, a diameter dimension is the second diameter dimension D2 smaller than the first diameter dimension D1. Therefore, the gap between the outer peripheral portion of the insertion section 5 and the intermediary duct defining portion 91 is reduced. However, the distal-side fixing portion 22 is made of a hard material. Therefore, even when the gap between the outer peripheral portion of the insertion section 5 and the intermediary duct defining portion 91 is small, frictional resistance between the outer peripheral portion of the insertion section 5 and the intermediary duct defining portion 91 does not increase. Therefore, the insertability of the insertion section 5 in the intermediary duct 92 and the removability of the insertion section 5 from the intermediary duct 92 can be assured. That is, the movability of the insertion section 5 in the intermediary duct 92 can be assured.

Since the mobility of the insertion section 5 in the endoscope insertion duct 37 and the intermediary duct 92 is assured, attachment of the endoscope cleaning sheath 3 to the endoscope 2 and removal of the endoscope cleaning sheath 3 from the endoscope 2 can be readily carried out. Furthermore, in the attachment of the endoscope cleaning sheath 3 to the endoscope 2 and the removal of the endoscope cleaning sheath 3 from the endoscope 2, a major part of movement of the insertion section 5 is movement in the endoscope insertion duct 37 having the large diameter dimension. Therefore, in the attachment of the endoscope cleaning sheath 3 to the endoscope 2 and the detachment of the endoscope cleaning sheath 3 from the endoscope 2, a distance that the insertion section 5 travels in the intermediary duct 92 having the small diameter dimension is small. Therefore, the attachment of the endoscope cleaning sheath 3 to the endoscope 2 and the removal of the endoscope cleaning sheath 3 from the endoscope 2 can be further easily carried out.

Additionally, in the state that the endoscope cleaning sheath 3 is attached to the endoscope 2, the bending section 9 of the insertion section 5 is placed to the proximal direction side with respect to the proximal end of the intermediary duct 92. That is, in the state that the endoscope cleaning sheath 3 is attached to the endoscope 2, the bending section 9 is placed in the multi-lumen tube 21 having the flexibility. Therefore, the multi-lumen tube 21 bends in accordance with a bending motion of the bending section 9. Therefore, an influence of the endoscope cleaning sheath 3 on the bending motion of the bending section 9 is reduced.

### (Modification of Sixth Embodiment)

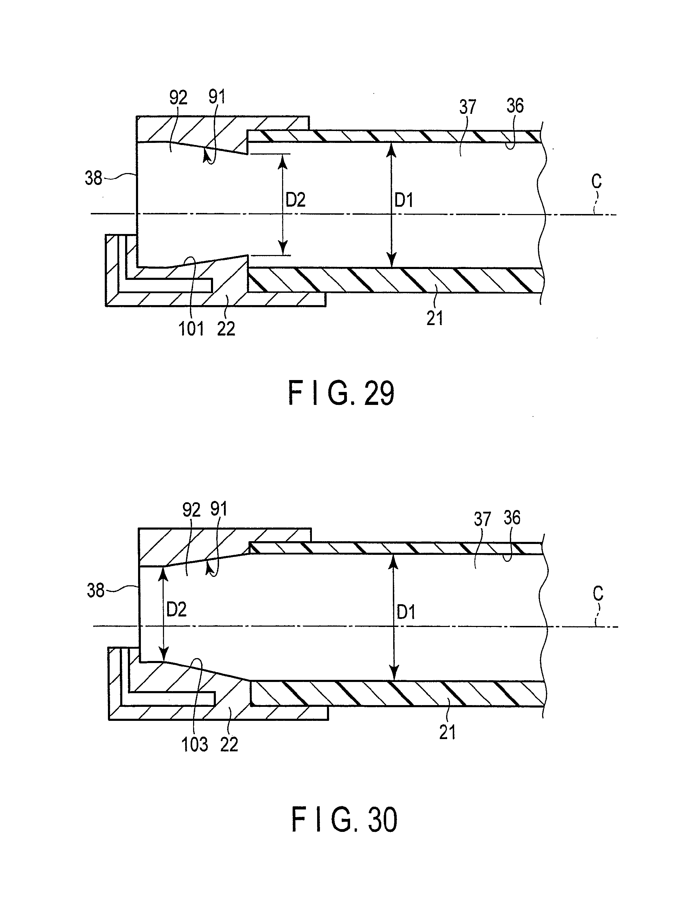

It is to be noted that, in the sixth embodiment, the diameter dimension of the intermediary duct 92 is the second diameter dimension D2 over the entire length in the directions parallel to the longitudinal axis C, but it is not restricted thereto. For example, as an 11th modification, a tapered surface 101 whose diameter dimension is reduced from the distal direction toward the proximal direction may be provided in the intermediary duct defining portion 91 as shown in FIG. 29. In this modification, at a proximal end of the tapered surface 101, a diameter dimension of the intermediary duct 92 is the second diameter dimension D2 smaller than the first diameter dimension D1 of the endoscope insertion duct 37. Therefore, at the proximal end of the tapered surface 101, in the state that the endoscope cleaning sheath 3 is attached to the endoscope 2, the gap between the outer peripheral portion of the insertion section 5 and the intermediary duct defining portion 91 is reduced. As a result, it is hard for the water to pass through the gap between the outer peripheral portion of the insertion section 5 and the intermediary duct defining portion 91 at the proximal end of the tapered surface 101, and the water can be effectively prevented from flowing into the gap between the insertion duct defining portion 36 and the outer peripheral portion of the insertion section 5 from the distal-side opening portion 38. Therefore, it is difficult for water to collect in the gap between the insertion duct defining portion 36 and the outer peripheral portion of the insertion section 5.

Additionally, for example, as a 12th modification, a tapered surface 103 whose diameter dimension is reduced from the proximal direction toward the distal direction may be provided in the intermediary duct defining portion 91 as shown in FIG. 30. In this modification, at a distal end of the tapered surface 103, a diameter dimension of the intermediary duct 92 is the second diameter dimension D2 smaller than the first diameter dimension D1 of the endoscope insertion duct 37. Therefore, at the distal end of the tapered surface 101, in the state that the endoscope cleaning sheath 3 is attached to the endoscope 2, the gap between the outer peripheral portion of the insertion section 5 and the intermediary duct defining portion 91 is small. As a result, at the distal end of the tapered surface 101, it is hard for water to pass through the gap between the outer peripheral portion of the insertion section 5 and the intermediary duct defining portion 91, and the water can be effectively prevented from entering the gap between the insertion duct defining portion 36 and the outer peripheral portion of the insertion section 5 from the distal-side opening portion 38. Therefore, it is difficult for water to collect in the gap between the insertion duct defining portion 36 and the outer peripheral portion of the insertion section 5.

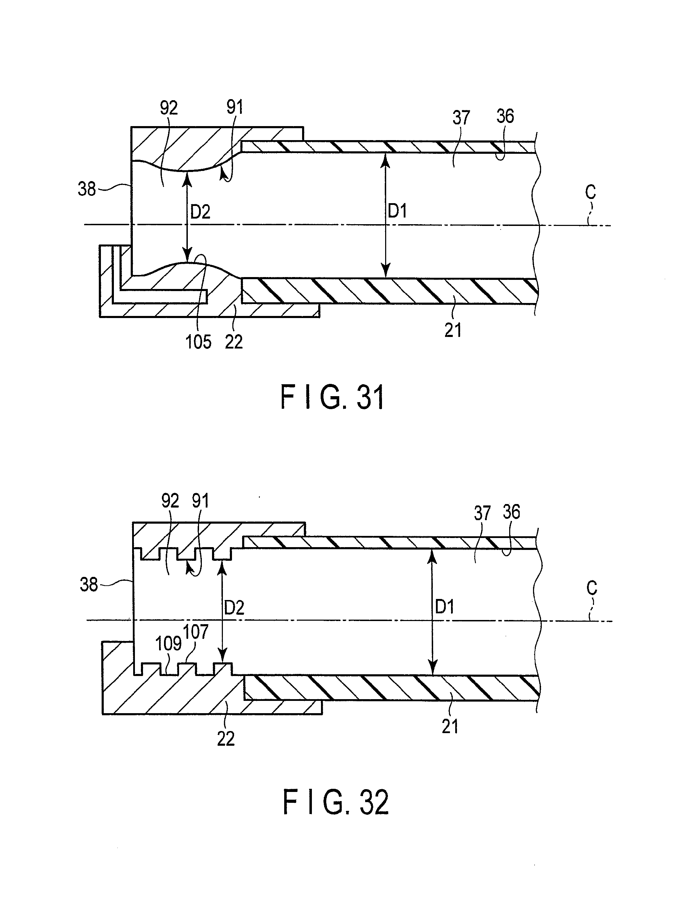

Further, for example, as a 13th modification, a protruding portion 105 that protrudes toward an inner peripheral direction may be provided on the intermediary duct defining portion 91 as shown in FIG. 31. The protruding portion 105 is formed into an arc shape in a cross section parallel to the longitudinal axis C. In this modification, at a protruding end of the protruding portion 105, a diameter dimension of the intermediary duct 92 is the second diameter dimension D2 smaller than the first diameter dimension D1 of the endoscope insertion duct 37. Therefore, at the protruding end of the protruding portion 105, in the state that the endoscope cleaning sheath 3 is attached to the endoscope 2, the gap between the outer peripheral portion of the insertion section 5 and the intermediary duct defining portion 91 is reduced. As a result, it is hard for water to pass through the gap between the outer peripheral portion of the insertion section 5 and the intermediary duct defining portion 91 at the protruding end of the protruding portion 105, and the water is effectively prevented from flowing into the gap between the insertion duct defining portion 36 and the outer peripheral portion of the insertion section 5 from the distal-side opening portion 38. Therefore, it is difficult for the water to collect in the gap between the insertion duct defining portion 36 and the outer peripheral portion of the insertion section 5.

Furthermore, for example, as a 14th modification, convex portions 107 and concave portions 109 may be alternately aligned and provided on the intermediary duct defining portion 91 in the directions parallel to the longitudinal axis C as shown in FIG. 32. In this modification, on each convex portion 107, a diameter dimension of the intermediary duct 92 is the second diameter dimension D2 smaller than the first diameter dimension D1 of the endoscope insertion duct 37. Therefore, at each convex portion 107, in the state that the endoscope sheath 3 is attached to the endoscope 2, the gap between the outer peripheral portion of the insertion section 5 and the intermediary duct defining portion 91 is reduced. As a result, at each convex portion 107, it is hard for water to pass through the gap between the outer peripheral portion of the insertion section 5 and the intermediary duct defining portion 91, and the water can be effectively prevented from flowing into the gap between the insertion duct defining portion 36 and the outer peripheral portion of the insertion section 5 from the distal-side opening portion 38. Therefore, it is difficult for the water to collect in the gap between the insertion duct defining portion 36 and the outer peripheral portion of the insertion section 5.

In the sixth embodiment and the 11th modification to the 14th modification, the intermediary duct defining portion 91 defines the diameter dimension of at least part of the intermediary duct 92 as the second diameter dimension D2 that is smaller than the first diameter dimension D1 of the endoscope insertion duct 37 and larger than the outer diameter dimension D0 of the insertion section 5. When such a configuration is adopted, it is hard for the water to pass through the gap between the outer peripheral portion of the insertion section 5 and the intermediary duct defining portion 91, and the water can be effectively prevented from flowing into the gap between the insertion duct defining portion 36 and the outer peripheral portion of the insertion section 5 from the distal-side opening portion 38. As a result, it is difficult for the water to collect in the gap between the insertion duct defining portion 36 and the outer peripheral portion of the insertion section 5.

Although the embodiments and the modifications of the present invention have been described above, the present invention is not restricted to the foregoing embodiments and modifications, and various modifications can be made without departing from the gist of the present invention as a matter of course.

## Claims

1. An endoscope cleaning sheath attached to an endoscope which includes an insertion section extended along a longitudinal axis, and in which an observation window is provided to a distal portion of the insertion section, the endoscope cleaning sheath comprising:
a sheath main body including: an insertion duct defining portion defining an endoscope insertion duct, which enables the insertion section of the endoscope to be inserted therein, along the longitudinal axis; and a fluid duct defining portion defining a fluid duct which enables a fluid to be supplied from a proximal direction toward a distal direction;
a distal-side fixing portion which is fixed to a distal portion of the sheath main body, and which includes a fluid emitting portion enabling emission of the fluid having passed through the fluid duct toward the observation window of the endoscope in a state that the endoscope cleaning sheath is attached to the endoscope;
a proximal-side fixing portion which is fixed to a proximal portion of the sheath main body; and
a linear portion which has a distal end fixed to the distal-side fixing portion at a distal-side connecting position, and a proximal end fixed to the proximal-side fixing portion at a proximal-side connecting position, and which is extended along a linear core axis between the distal-side connecting position and the proximal-side connecting position, the linear portion having a higher tensile strength in directions parallel to the longitudinal axis than the sheath main body.

2. The endoscope cleaning sheath according to claim 1,
wherein a linear dimension of the linear portion along the linear core axis between the distal-side connecting position and the proximal-side connecting position is larger than a parallel axial dimension along the longitudinal axis between the distal-side connecting position and the proximal-side connecting position.

3. The endoscope cleaning sheath according to claim 2,
wherein the sheath main body includes: a first hole defining portion defining a first hole through which the endoscope insertion duct communicates with an outside of the sheath main body; and a second hole defining portion defining a second hole through which the endoscope insertion duct communicates with the outside of the sheath main body separately from the first hole, and
the linear portion is extended through the endoscope insertion duct between the distal-side connecting position and the proximal-side connecting position, extended from the endoscope insertion duct to the outside of the sheath main body in the first hole, and inserted into the endoscope insertion duct from the outside of the sheath main body in the second hole.

4. The endoscope cleaning sheath according to claim 3,
wherein the first hole and the second hole are apart from each other in the directions parallel to the longitudinal axis, and
the linear portion includes a loop-shaped portion which is formed between the first hole and the second hole.

5. The endoscope cleaning sheath according to claim 4,
wherein the first hole is placed to a proximal direction side with respect to the second hole, and
the linear portion is extended toward the proximal direction in the endoscope insertion duct until it reaches the first hole, passes through the first hole and the second hole, and is extended toward the proximal direction from the second hole in the endoscope insertion duct.

6. The endoscope cleaning sheath according to claim 4,
wherein the linear portion passes through the first hole and the second hole from the endoscope insertion duct and is again extended to the outside of the sheath main body through the first through hole.

7. The endoscope cleaning sheath according to claim 2,
wherein the sheath main body includes a hole defining portion defining a hole through which the endoscope insertion duct communicates with an outside of the sheath main body,
the linear portion is extended through the endoscope insertion duct between the distal-side connecting position and the proximal-side connecting position, and
the linear portion includes: an external extended portion which is extended from the endoscope insertion duct to the outside of the sheath main body in the hole; a duct inserting portion which is inserted into the endoscope insertion duct from the outside of the sheath main body in the hole; and a circumferential extended portion which is extended along circumferential directions of the sheath main body between the external extended portion and the duct inserting portion.

8. The endoscope cleaning sheath according to claim 7,
wherein the external extended portion of the linear portion is hitched on the duct inserting portion in the hole.

9. The endoscope cleaning sheath according to claim 2,
wherein the linear portion is extended from the distal-side connecting portion to the proximal-side connecting portion on an outer peripheral portion of the sheath main body.

10. The endoscope cleaning sheath according to claim 9,
wherein the linear portion includes a spiral portion which is spirally extended in the directions parallel to the longitudinal axis.

11. The endoscope cleaning sheath according to claim 2,
wherein the fluid duct is separated from the endoscope insertion duct by the fluid duct defining portion and the insertion duct defining portion, and
the linear portion is extended from the distal-side connecting position to the proximal-side connecting position in the fluid duct.

12. The endoscope cleaning sheath according to claim 2,
wherein the sheath main body includes a separation duct defining portion defining a separation duct in a state that the separation duct is separated from the endoscope insertion duct and the fluid duct, and
the linear portion is extended from the distal-side connecting position and the proximal-side connecting position in the separation duct.

13. The endoscope cleaning sheath according to claim 1,
wherein the fluid emitting portion is configured to emit the fluid from a first perpendicular direction perpendicular to the longitudinal axis toward a second perpendicular direction that is a direction opposite to the first perpendicular direction, and also configured to emit the fluid from an emission port placed at a second-perpendicular-direction-side end thereof, and
the distal-side connecting position of the linear portion is placed to a second perpendicular direction side with respect to the emission port.

14. An endoscope device comprising:
the endoscope cleaning sheath according to claim 1; and
the endoscope which includes the insertion section which is extended along the longitudinal axis, and which is configured to be inserted into the endoscope insertion duct of the sheath main body, the endoscope cleaning sheath being attached to the endoscope, the insertion section including the observation window in the distal portion thereof.

15. The endoscope device according to claim 14,
wherein the insertion section includes a bending section configured to bend in a bending direction perpendicular to the longitudinal axis,
the sheath main body is configured to bend in accordance with a bending motion of the bending section, and
a linear dimension of the linear portion along the linear core axis between the distal-side connecting position and the proximal-side connecting position is larger than a parallel axial dimension along the longitudinal axis between the distal-side connecting position and the proximal-side connecting position.

16. The endoscope device according to claim 15,
wherein the sheath main body includes: a first hole defining portion defining a first hole through which the endoscope insertion duct communicates with an outside of the sheath main body; and a second hole defining portion defining a second hole through which the endoscope insertion duct communicates with the outside of the sheath main body separately from the first hole, and
the linear portion is extended through the endoscope insertion duct between the distal-side connecting position and the proximal-side connecting position, extended from the endoscope insertion duct to the outside of the sheath main body in the first hole, and inserted into the endoscope insertion duct from the outside of the sheath main body in the second hole.

17. The endoscope device according to claim 15,
wherein the linear portion is placed at an angular position deviating from the bending direction of the bending section in directions around the longitudinal axis.

18. An endoscope cleaning sheath attached to an endoscope which includes an insertion section extended along a longitudinal axis, and in which an observation window provided to a distal portion of the insertion section, the endoscope cleaning sheath comprising:
a sheath main body which is extended along the longitudinal axis, and in which a cross section perpendicular to the longitudinal axis is formed into the same shape over an entire length in directions parallel to the longitudinal axis, the sheath main body including a fluid duct defining portion defining a fluid duct through which supply of a fluid from a proximal direction toward a distal direction is enabled;
a distal-side fixing portion which is fixed to a distal portion of the sheath main body, and which is made of a material harder than the sheath main body, the distal-side fixing portion including a fluid emitting portion which enables emitting the fluid having passed through the fluid duct toward the observation window of the endoscope in a state that the endoscope cleaning sheath is attached to the endoscope;
an insertion duct defining portion defining an endoscope insertion duct, which enables the insertion section of the endoscope to be inserted therein, in the sheath main body along the longitudinal axis, and also defining a diameter dimension of the endoscope insertion duct as a first diameter dimension over the entire length in the directions parallel to the longitudinal axis; and
an intermediary duct defining portion defining in the distal-side fixing portion an intermediary duct which has a proximal end communicating with a distal end of the endoscope insertion duct, and also defining the intermediary duct in a state that a distal end of the intermediary duct is opened with respect to an outside of the distal-side fixing portion, the intermediary duct defining portion defining a diameter dimension of at least part of the intermediary duct as a second diameter dimension that is smaller than the first diameter dimension and larger than an outer diameter dimension of the insertion section.

19. The endoscope cleaning sheath according to claim 18,
wherein a difference between the outer diameter dimension of the insertion section and the second diameter dimension is 0.1 mm or less.

20. The endoscope cleaning sheath according to claim 18, further comprising a water-shedding coating which coats the intermediary duct defining portion.

21. An endoscope device comprising:
the endoscope cleaning sheath according to claim 18; and
the endoscope including the insertion section which is extended along the longitudinal axis, and which is configured to be inserted into the endoscope insertion duct of the sheath main body, the endoscope cleaning sheath being attached to the endoscope, the insertion section including the observation window in the distal portion thereof.

22. The endoscope device according to claim 21,
wherein the insertion section includes a bending section configured to bend in a bending direction perpendicular to the longitudinal axis, and
the bending section is placed to a proximal direction side with respect to the proximal end of the intermediary duct in a state that the endoscope cleaning sheath is attached to the endoscope.
